# EUROPEAN PATENT APPLICATION

(11) **EP 2 399 618 A1**
(43) Date of publication of application: **28.12.2011**
(21) Application number: 10166811.9
(22) Date of filing: 22.06.2010
(51) Int. Cl.: A61L 27/34, A61L 27/54, A61F 2/82, A61F 2/02, A61L 31/08

(54) **Antimicrobial medical devices**

(71) Applicant: Planton GmbH, 24106 Kiel (DE)
(72) Inventor: Kleine, Michael, 24159, Kiel (DE); Weigel, Martin Christian, 24145, Kiel (DE); Hofmann-Peiker, Karsten, 24111, Kiel (DE)
(74) Representative: Dehmel, Albrecht

(57) **Abstract**

The present invention relates to a medical device having a silane surface comprising an antimicrobial peptide, wherein the antimicrobial peptide is attached to the silane surface.

SEQUENCE LISTING

<110>  PLANTON GmbH

<120>  Antimicrobial medical devices

<130>  PLA-014

<160>  12

<170>  PatentIn version 3.3

<210>  1
<211>  41
<212>  PRT
<213>  human

<400>  1

Gly Ile Gly Asp Pro Val Thr Cys Leu Lys Ser Gly Ala Ile Cys His
1               5                   10                  15

Pro Val Phe Cys Pro Arg Arg Tyr Lys Gln Ile Gly Thr Cys Gly Leu
            20                  25                  30

Pro Gly Thr Lys Cys Cys Lys Lys Pro
        35                  40

<210>  2
<211>  126
<212>  DNA
<213>  human

<400>  2
ggtataggcg atcctgttac ctgccttaag agtggagcca tatgtcatcc agtcttttgc      60

cctagaaggt ataaacaaat tggcacctgt ggtctccctg gaacaaaatg ctgcaaaaag     120

ccatga                                                                126

<210>  3
<211>  126
<212>  DNA
<213>  artificial

<220>
<223>  hbd-2 optimized codon usage pattern

<400>  3
ggaataggcg atcctgttac ctgtcttaaa agtggagcca tatgtcatcc tgtcttctgt      60

cctaggaggt ataagcaaat aggcacctgt ggactccctg gaacaaaatg ttgtaaaaaa     120

ccttga                                                                126

<210>  4
<211>  45
<212>  PRT
<213>  human

<400>  4

Gly Ile Ile Asn Thr Leu Gln Lys Tyr Tyr Cys Arg Val Arg Gly Gly

1               5                   10                  15

Arg Cys Ala Val Leu Ser Cys Leu Pro Lys Glu Glu Gln Ile Gly Lys
            20                  25                  30

Cys Ser Thr Arg Gly Arg Lys Cys Cys Arg Arg Lys Lys
        35                  40                  45

<210>  5
<211>  138
<212>  DNA
<213>  human

<400>  5
ggaatcataa acacattaca gaaatattat tgcagagtca gaggcggccg gtgtgctgtg      60

ctcagctgcc ttccaaagga ggaacagatc ggaaagtgct cgacgcgtgg ccgaaaatgc     120

tgccgaagaa agaaataa                                                   138

<210>  6
<211>  138
<212>  DNA
<213>  artificial

<220>
<223>  hbd-3 optimized codon usage pattern

<400>  6
ggaatcataa atacacttca gaaatattat tgcagagtta gaggaggaag atgtgctgtg      60

cttagctgcc ttccaaagga ggaacagatc ggaaagtgct ctactcgtgg aagaaaatgc     120

tgcagaagaa agaaataa                                                   138

<210>  7
<211>  128
<212>  PRT
<213>  human

<400>  7

Lys Pro Lys Gly Met Thr Ser Ser Gln Trp Phe Lys Ile Gln His Met
1               5                   10                  15

Gln Pro Ser Pro Gln Ala Cys Asn Ser Ala Met Lys Asn Ile Asn Lys
            20                  25                  30

His Thr Lys Arg Cys Lys Asp Leu Asn Thr Phe Leu His Glu Pro Phe
        35                  40                  45

Ser Ser Val Ala Ala Thr Cys Gln Thr Pro Lys Ile Ala Cys Lys Asn
    50                  55                  60

Gly Asp Lys Asn Cys His Gln Ser His Gly Pro Val Ser Leu Thr Met
65                  70                  75                  80

Cys Lys Leu Thr Ser Gly Lys Tyr Pro Asn Cys Arg Tyr Lys Glu Lys

                85                  90                  95

Arg Gln Asn Lys Ser Tyr Val Val Ala Cys Lys Pro Pro Gln Lys Lys
            100                 105                 110

Asp Ser Gln Gln Phe His Leu Val Pro Val His Leu Asp Arg Val Leu
        115                 120                 125

<210> 8
<211> 387
<212> DNA
<213> human

<400> 8
aagcccaagg gcatgacctc atcacagtgg tttaaaattc agcacatgca gcccagccct 60

caagcatgca actcagccat gaaaaacatt aacaagcaca caaaacggtg caaagacctc 120

aacaccttcc tgcacgagcc tttctccagt gtggccgcca cctgccagac ccccaaaata 180

gcctgcaaga atggcgataa aaactgccac cagagccacg ggcccgtgtc cctgaccatg 240

tgtaagctca cctcagggaa gtatccgaac tgcaggtaca aagagaagcg acagaacaag 300

tcttacgtag tggcctgtaa gcctccccag aaaaaggact ctcagcaatt ccacctggtt 360

cctgtacact tggacagagt cctttag 387

<210> 9
<211> 387
<212> DNA
<213> artificial

<220>
<223> RNAse 7 optimized codon usage pattern

<400> 9
aagcccaagg gcatgacctc ttctcaatgg ttcaaaattc aacatatgca acccagccct 60

caagcttgta attctgccat gaaaaatatt aataagcata caaaacggtg taaggatctc 120

aataccttcc ttcatgaacc tttctccagt gtggccgcca cctgtcaaac ccccaaaata 180

gcctgtaaga atggcgataa aaattgtcat caaagccatg gacccgtgtc ccttaccatg 240

tgtaagctca cctctggaaa gtatccgaat tgtaggtaca aagaaaagcg acaaaataag 300

tcttacgtag tggcctgtaa gcctccccaa aaaaaggatt ctcaacaatt ccatcttgtt 360

cctgtacatt tggatagggt tctttag 387

<210> 10
<211> 128
<212> PRT
<213> artificial

<220>
<223> RNAse7 derivative optimized codon usage pattern

<400> 10

Lys Pro Lys Gly Met Thr Ser Ser Gln Trp Phe Lys Ile Gln His Met
1               5                   10                  15

Gln Pro Ser Pro Gln Ala Cys Asn Ser Ala Met Lys Asn Ile Asn Lys
            20                  25                  30

His Thr Lys Arg Cys Arg Asp Leu Asn Thr Phe Leu His Glu Pro Phe
        35                  40                  45

Ser Ser Val Ala Ala Thr Cys Gln Thr Pro Lys Ile Ala Cys Lys Asn
    50                  55                  60

Gly Asp Lys Asn Cys His Gln Ser His Gly Pro Val Ser Leu Thr Met
65                  70                  75                  80

Cys Lys Leu Thr Ser Gly Lys Tyr Pro Asn Cys Arg Tyr Lys Glu Lys
                85                  90                  95

Arg Gln Asn Lys Ser Tyr Val Val Ala Cys Lys Pro Pro Gln Lys Lys
            100                 105                 110

Asp Ser Gln Gln Phe His Leu Val Pro Val Asp Leu Asp Arg Val Leu
        115                 120                 125

<210>  11
<211>  387
<212>  DNA
<213>  artificial

<220>
<223>  RNAse7 derivative optimized codon usage pattern

<400>  11
aagcccaagg gcatgacctc atcacagtgg tttaaaattc agcacatgca gcccagccct      60

caagcatgca actcagccat gaaaaacatt aacaagcaca caaaacggtg cagagacctc     120

aacaccttcc tgcacgagcc tttctccagt gtggccgcca cctgccagac ccccaaaata     180

gcctgcaaga atggcgataa aaactgccac cagagccacg ggcccgtgtc cctgaccatg     240

tgtaagctca cctcagggaa gtatccgaac tgcaggtaca aagagaagcg acagaacaag     300

tcttacgtag tggcctgtaa gcctccccag aaaaaggact ctcagcaatt ccacctggtt     360

cctgtagact tggacagagt cctttag                                         387

<210>  12
<211>  387
<212>  DNA
<213>  artificial

<220>
<223>  RNAse7 derivative optimized codon usage pattern

<400>  12
aagcccaagg gcatgacctc ttctcaatgg ttcaaaattc aacatatgca acccagccct      60

caagcttgta attctgccat gaaaaatatt aataagcata caaaacggtg tagggatctc     120

aataccttcc ttcatgaacc tttctccagt gtggccgcca cctgtcaaac ccccaaaata     180

gcctgtaaga atggcgataa aaattgtcat caaagccatg gacccgtgtc ccttaccatg 240
tgtaagctca cctctggaaa gtatccgaat tgtaggtaca aagaaaagcg acaaaataag 300
tcttacgtag tggcctgtaa gcctccccaa aaaaaggatt ctcaacaatt ccatcttgtt 360
cctgtagatt tggatagggt tctttag 387

## Description

The present invention relates to a medical device having a silane surface comprising an antimicrobial peptide, wherein the antimicrobial peptide is attached to the silane surface.

The use of titanium and its alloys in medical applications has increased significantly in recent years. Since the 1970s this biomaterial is well-accepted and can be considered as the material of choice for artificial endosseous implants so far.

Today, as a result of intraoperative bacterial contamination during the course of implant insertion combined with surgical tissue destruction peri-implant infections of bone and its surrounding tissue are a common postoperative complication. The long term survival of implants depends mostly on the control of bacterial infections in the peri-implant region and its functional stability. The gram-positive bacteria *Staphylococcus aureus* and several other strains of the genus *Staphylococcus* are frequently associated with the colonization of metallic orthopaedic implants and are responsible for subsequent infections, and it has been demonstrated that this bacterium has the ability to adhere to titanium surfaces.

The infection is often dependent on the microflora of the peri-implant environment within the human body. Especially hospital-acquired multidrug-resistant bacteria causing these severe infections play an increasing role. Antibiotics like covalently attached vancomycine onto titanium surfaces reduced colony-forming of gram-positive bacteria up to 88% in vitro.

However, the use of prophylactic local antibiotics during implant placement remains controversial. On the one hand infections around biomaterials are difficult to treat and almost all infected implants have to be removed at one stage. On the other hand prophylactic treatment of classical antibiotics may trigger allergic reactions, and, more intriguing, will support the selection of severe antibiotic resistant bacteria.

Thus, there is a great interest in the development of surfaces and coatings that can actively kill micro-organisms. Probably the oldest and most widespread coatings are silver ions, successfully applied against methicillin resistant *Staphylococcus aureus* (MRSA). However, a drawback of this approach is the cytotoxicity of silver ions towards mammalian cells.

Antimicrobial peptides (AMPs), which have been isolated from many bacteria, fungi, plants, invertebrates and vertebrates are an important component of the natural defenses of most living organisms. AMPs represent a wide range of short, gene-encoded peptide antibiotics, but also antivirals, templates for cell-penetrating peptides, immunomodulators and anti tumoural drugs. These peptides show variable activity against invading pathogens and build an integral component of the innate immune response that the skin uses to respond and prevent the uncontrolled growth of micro-organisms. AMPs have demonstrated in studies to kill *S. aureus,* herpes simplex virus, vaccinia virus and the *Malassezia* species pathogenic micro-organisms associated with significant morbidity in patients with atopic dermatitis (AD) (Ong et al., 2002).

The object of the present invention is to provide a medical device with antimicrobial activity.

The object is solved by the subject matter as defined in the claims.

The following figures illustrate the present invention.

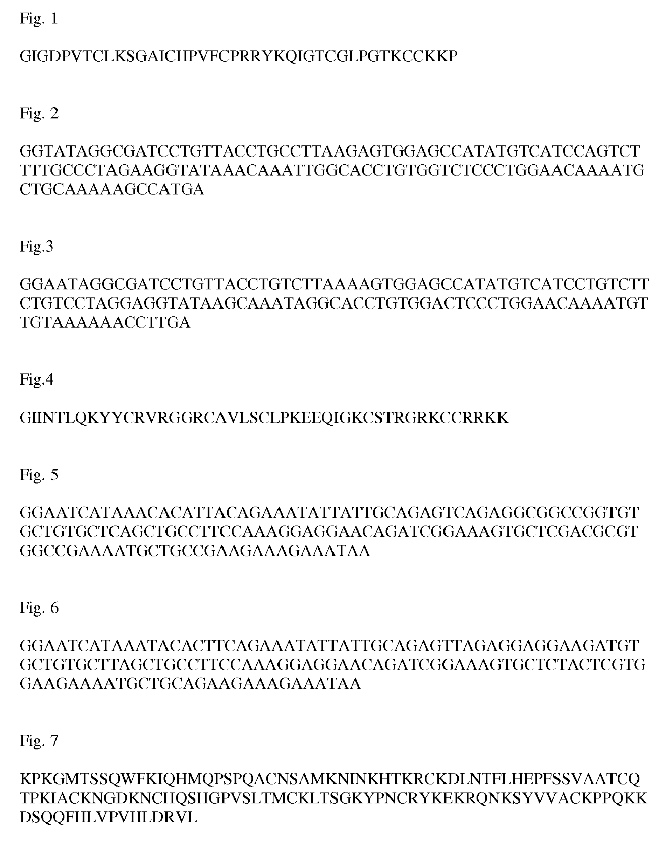

Figure 1 shows the amino acid sequence (SEQ ID NO: 1) of human 13-defensin-2 (hpD2).
Figure 2 shows the nucleic acid sequence (SEQ ID NO: 2) encoding the amino acid sequence according to SEQ ID NO: 1.
Figure 3 shows the nucleic acid sequence with optimized codon usage pattern (SEQ ID NO: 3.) for high-level expression of recombinant hBD2 in plants.
Figure 4 shows the amino acid sequence (SEQ ID NO: 4) of human B-defensin-3 (h13D3).
Figure 5 shows the nucleic acid sequence (SEQ ID NO: 5) encoding the amino acid sequence according to SEQ ID NO: 4.
Figure 6 shows the nucleic acid sequence with optimized codon usage pattern (SEQ ID NO: 6) for high-level expression of recombinant hβD3 in plants.
Figure 7 shows the amino acid sequence (SEQ ID NO: 7) of Ribonuclease 7 (RNAse 7).

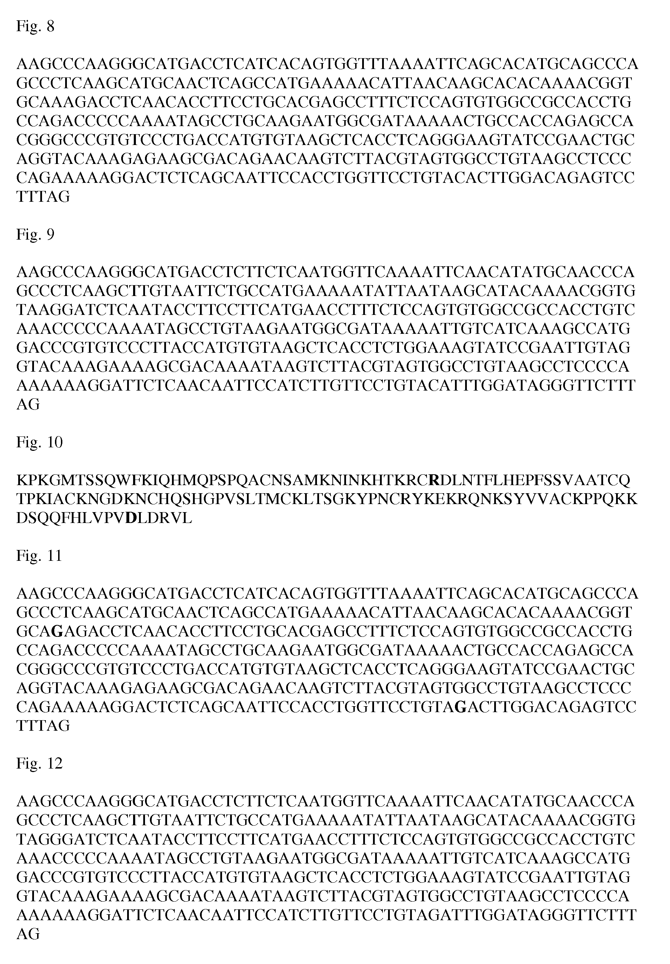

Figure 8 shows the nucleic acid sequence (SEQ ID NO: 8) encoding the amino acid sequence according to SEQ ID NO: 7.
Figure 9 shows the nucleic acid sequence with optimized codon usage pattern (SEQ ID NO: 9) for high-level expression of recombinant RNAse 7 in plants.
Figure 10 shows the amino acid sequence (SEQ ID NO: 10) of a Ribonuclease 7 (RNAse 7) derivative.
Figure 11 shows the nucleic acid sequence (SEQ ID NO: 11) encoding the amino acid sequence according to SEQ ID NO: 10.
Figure 12 shows the nucleic acid sequence with optimized codon usage pattern (SEQ ID NO: 12) for high-level expression of recombinant RNAse 7 in plants.
Figure 13 shows a bar chart representing the killing activity (0 % to 100%) of 80 pg hpD2 adsorbed by Hexadecyltrimethoxysilane (SAM 1, black bars), Dimethoxymethyloctylsilane (SAM 2, fasciated bars), and oxidized Allyltrimethoxysilane (SAM 3, white bars) on modified Titanium pins after 2, 4, 6 and 8 hours of cultivation against E. *coli.* The mean of five independent repetitions is shown. Error bars show standard deviation of the mean.

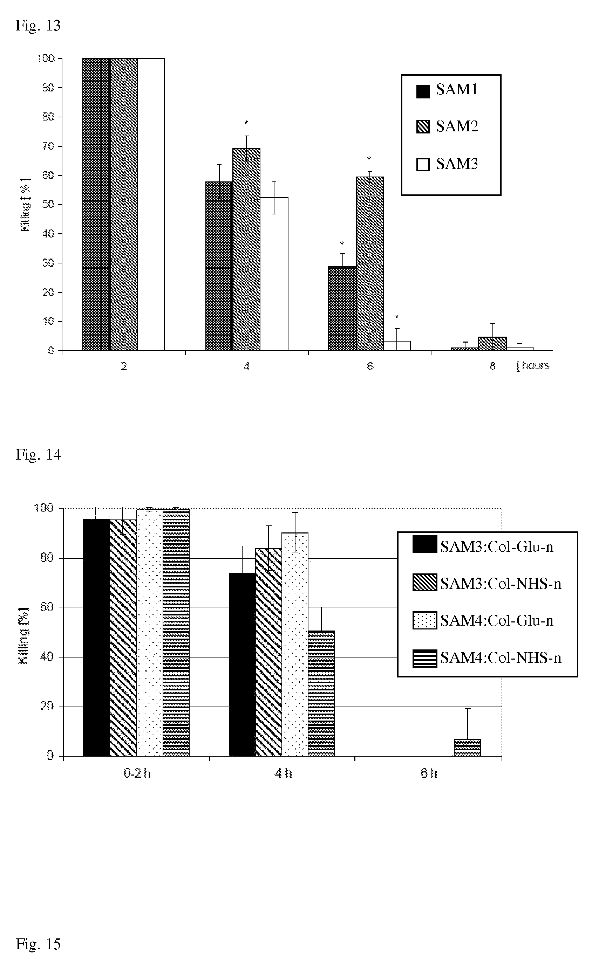

Figure 14 shows a bar chart representing the killing activity (0 % to 100%) of 80 µg hβD2 coated on collagen functionalized titanium pins with different cross-linking strategies in comparison to pins without hpD2 after 2, 4 and 6 hours of culturing against *E*. *coli.* Collagen cross-linking to Allyltrimethoxysilane (SAM 3) and to 3-Aminopropyltrimethoxysilane (SAM 4) surface by application of glutaraldehyde (SAM 3 black bars: SAM3:Col-Glu-n and SAM 4 dotted bars: SAM4:Col-Glu-n) and NHS/EDC (SAM 3 fasciated bars: SAM3:Col-NHS-n and SAM 4 horizontal striped: SAM4:Col-NHS-n). The mean of five independent repetitions is shown. Error bars show standard deviation of the mean.
Figure 15 shows a bar chart representing the killing activity (0% to 100%) of different amounts of directly applied hpD2 (S1 to S5) in comparison with the killing activity of different hpD2 coated biopolymers (P1 to P6) in a diffusion assay against *E. coli.* S1 to S5 shows the activity of directly applied hpD2: S 1: 0.01 % acetic acid; S2: 0.1 ug HBD2; S3: 1 ug HBD2; S4: 5 µg HBD2; S5: 10 ug HBD2; P1 to P5 shows the activity of biopolymers treated with 10 ug hβD2: P1: hyaluronic acid, P2: alginic acid; P3: gelatine; P4: agarose (2 variants); P5: polylactide matrix (three variants); P6: collagen scaffold. The chosen biopolymers show no self-activity (Data not shown).

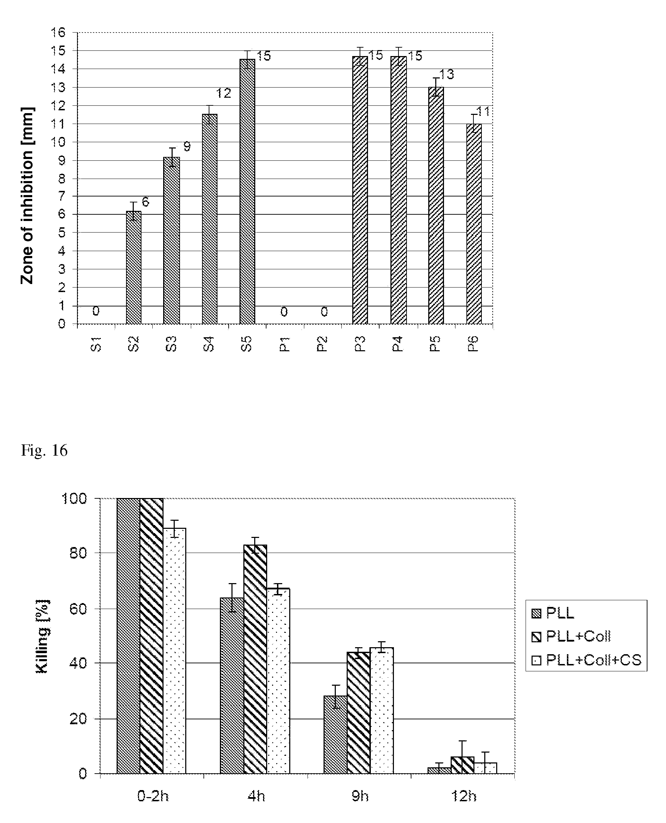

Figure 16 shows a bar chart representing the killing activity (0% to 100%) of 25 ug hβD2 coated on polylactide matrices after 0-2, 4, 9 and 12 hours of culturing against E *coli* in a microdilution assay, wherein the polylactide matrices are coated additionally with collagen (PLL+Koll), with collagen and chondroitin sulphate (PLL+Koll+CS) in comparison with not additionally coated matrices (PLL). All polylactide matrices without hpD2 show no self-activity.

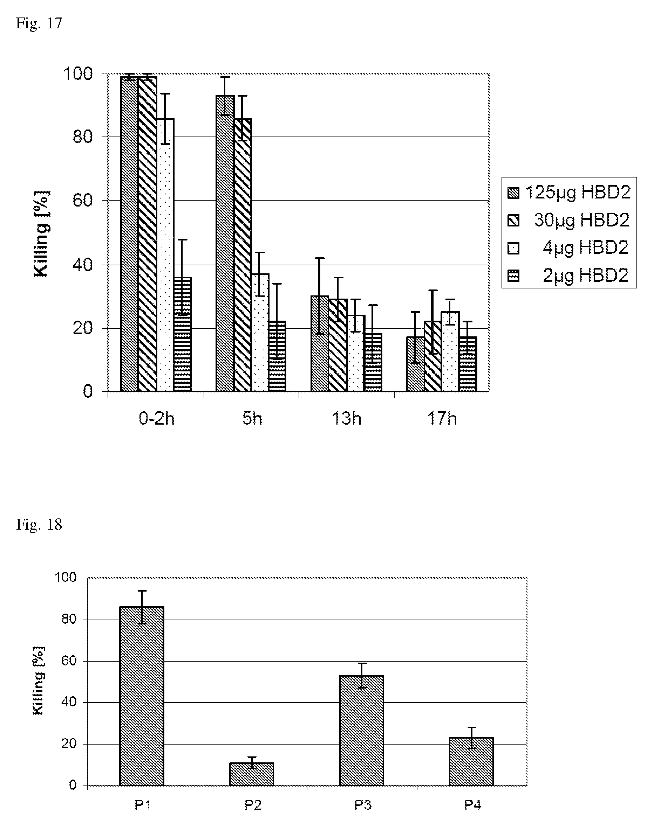

Figure 17 shows a bar chart representing the killing activity (0% to 100%) of collagen scaffolds coated with different hβD2 amounts (2, 4, 30 and 125 ug) after 0-2, 5, 13 and 17 hours of culturing against *E*. *coli* in a microdilution assay.
Figure 18 shows a bar chart representing the killing activity (0% to 100%) of collagen scaffolds blocked with different amino acids (P2: L-lysine; P3: L-glutamic acid; P4: poly-L-glutamic acid) in comparison with the activity of unblocked collagen scaffold (P1), wherein the collagen scaffolds are additionally coated with 4 ug hβD2 after 0-2 hours of culturing against E. *coli* in a microdilution assay. The amino acids have a self-activity of 4 %.

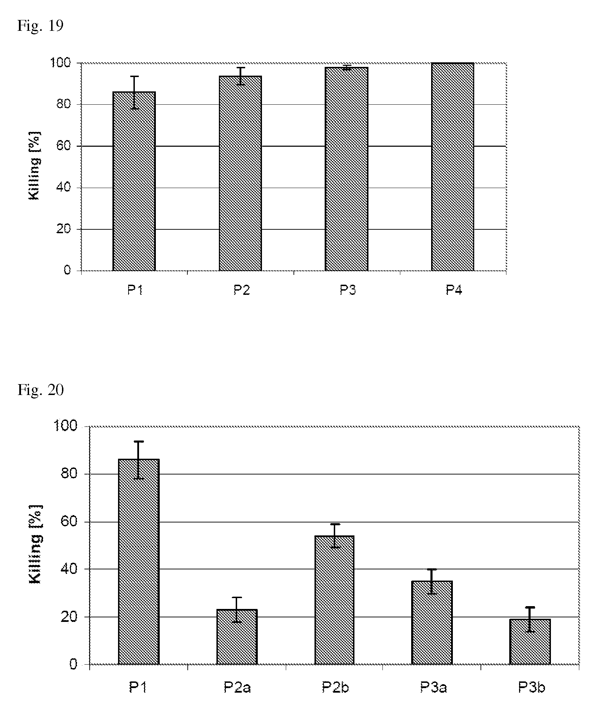

Figure 19 shows a bar chart representing the killing activity (0% to 100%) of collagen scaffolds blocked with different proteins (P2: gelatine; P3: bovine serum albumin (BSA); P4: human serum albumin (HAS)) in comparison with the activity of unblocked collagen scaffold (P1), wherein the collagen scaffolds are additionally coated with 4 ug hβD2 after 0-2 hours of culturing against E. *coli* in a microdilution assay. The proteins show no self-activity (data not shown).
Figure 20 shows a bar chart representing the killing activity (0% to 100%) of collagen scaffolds blocked with further substances (P2a: 5 ug chondroitin sulphate; P3a: 20 µg spermidine) in comparison with the activity of unblocked collagen scaffold (P1), wherein some collagen scaffolds are additionally coated with 4 ug hβD2 (P2b: 5 ug chondroitin sulphate and 4 µg hβD2; P3b: 20 µg spermidine and 4 ug hβD2) after 0-2 hours of culturing against E. *coli* in a microdilution assay.

The term "antimicrobial peptide" as used herein refers to a peptide which inhibits and/or kills pathogenic micro-organisms, for example bacteria, viruses, fungi, yeasts, mycoplasma and protozoa. The antimicrobial peptide may be a member of the RNAse A super family, a defensin, cathelicidin, granulysin, histatin, psoriasin, dermicidine or hepzidine. Members of the RNAse A super family are for example RNAse 1, RNAse 2, RNAse 3, RNAse 4, RNAse 5, RNAse 6, RNAse 7, RNAse 8 and RNAse 9. Defensins including for example alpha- and beta-defensins, like human beta-defensin-1, human beta-defensin-2, human beta-defensin-3, human beta-defensin-4, human alpha-defensin 1, human alpha-defensin-2, human alpha-defensin-3, human alpha-defensin-4, human alpha-defensin-5 and human alpha-defensin-6. Cathelicidin may be for example LL-37. Histatins are for example histatin 1, histatin 2, histatin 3, histatin 4, histatin 5, histatin 6 and histatin 7. Hepzidin may be for example hepzidin-20 or hepzidin-25. The antimicrobial peptide may be naturally occurring in insect, fish, plant or mammalian cells, preferably human cells.

The term "human β-defensin-2" or "hβD2" as used herein refers to a polypeptide with an antimicrobial effect. Preferably human p-defensin-2 exhibits the amino acid sequence according to SEQ ID NO: 1. More preferably human β-defensin-2 is encoded by the nucleic acid according to SEQ ID NO: 2. Even more preferably human β-defensin-2 is encoded by a nucleic acid with optimized codon usage pattern. Most preferably human β-defensin-2 is encoded by the nucleic acid according to SEQ ID NO: 3. The human β-defensin-2 naturally occurs in human epithelial cells of the skin and the respiratory, urogenital and gastrointestinal tract.

The term "human β-defensin-3" or "hβD3" as used herein refers to a polypeptide with an antimicrobial effect. Preferably human β-defensin-3 exhibits the amino acid sequence according to SEQ ID NO: 4. More preferably human β-defensin-3 is encoded by the nucleic acid according to SEQ ID NO: 5. Even more preferably human β-defensin-3 is encoded by a nucleic acid with optimized codon usage pattern. Most preferably human β-defensin-3 is encoded by the nucleic acid according to SEQ ID NO: 6. The human β-defensin-3 naturally occurs in human epithelial cells of the skin and the respiratory, urogenital and gastrointestinal tract.

The term "Ribonuclease 7" or "RNAse 7" as used herein refers to a polypeptide with an antimicrobial effect. Preferably Ribonuclease 7 exhibits the amino acid sequence according to SEQ ID NO: 7. More preferably Ribonuclease 7 is encoded by the nucleic acid according to SEQ ID NO: 8. Even more preferably Ribonuclease 7 is encoded by a nucleic acid with optimized codon usage pattern. Most preferably Ribonuclease 7 is encoded by the nucleic acid according to SEQ ID NO: 9. Ribonuclease 7 naturally occurs in human epithelial tissues including skin, respiratory tract, genitourinary tract and gut. Preferably, RNAse 7 naturally occurs in human keratinocytes.

The term "optimized codon usage pattern", as used herein to refer to the substitution of some of the codons coding for a amino acid of interest with such codons as to increase the expression level of the protein of interest in cells or tissues in different organisms. Various combinations of the codons to be substituted can be applied for achieving the increase in the expression level by those skilled in the art.

The term "recombinant" as used herein refers to a peptide expressed in any other organism or cell culture than in the natural source. Other organism includes, but is not limited to plants, bacteria, yeast and fungi. The cell culture includes, but is not limited to human, mammalian, insect and plant cell culture.

The term "synthetic" refers to a peptide obtained by connecting one amino acid with another by forming a peptide bond. Methods for the production of synthetic peptides are for example the solid phase peptide synthesis (SPPS) and the liquid phase peptide synthesis (LPPS).

The term "antimicrobial" as used herein refers to the activity against any endogenous or exogenous organisms causing disease. Disease causing organisms include, but are not limited to, bacteria, viruses, yeast, protozoa, fungi, or any combination or derivative thereof. Viruses include but are not limited to adenovirus, papilloma virus, human immunodifficiency virus and the human herpes simplex virus. Bacteria include, but are not limited to, gram-positive and gram-negative bacteria, in particular *Acinetobacter baumannii, Escherichia coli, Klebsiella pneumoniae, Pseudomonas aeruginosa, Salmonella typhimurium, Propionibacterium acnes, Staphylococcus aureus, Staphylococcus epidermidis, Streptococcus pyogenes and Streptococcus pneumoniae.*

The term "medical device" as used herein refers to any type of appliance that is totally or partly introduced, surgically or medically, into a patient's body and which may remain there after the procedure or may be removed during the treatment. The duration of implantation may be essentially permanent, i.e., intended to remain in place for the remaining lifespan of the patient or until it is physically removed. Examples of medical devices include, without limitation, implants, instruments, sutures, carriers, dressings, viscoelastica and replacement body parts. The implants include for example orthopedic and dental implants. Orthopedic implants may be for example plates, in particular bone plates, screws, in particular bone screws, pins, wires, femoral heads, nails, in particular hip nails, intramedullary interlocking nails and/or Kuntscher cloverleaf nails. The instrument may be a surgical instrument. Surgical instruments include, but are not limited to, instruments for bone screws, for bone plates, for pins, for wires, for femoral heads, for hip nails, for knee surgery, for skull surgery, retractors, elevators, hooks and levers, in particular Bennett's, hook for skin with three prong, skin hook-2 prong, cobs elevator, dental elevator L/R and cryer elevator; bone cutting instruments, forceps, chisels, osteotomes, gouges and curpets. The sutures may be, but are not limited to surgical sutures, which may be absorbable or non-absorbable. Implants may also be heart valves, ligaments, sinews, vasculatures, pericardium, temporary filling material of the skin matrix and replacement vitreous body. The medical device may be of, but is not limited to, gold, silver, stainless steel, titanium, gelatine, agarose, collagen, polylactide, hyaluronic acid and alginic acid.

The term "self-assembled monolayer" as used herein refers to a molecule that has one or more chemical groups which attach to a surface strongly, wherein a portion of the molecule will bind to one or more neighbouring self-assembled monolayer molecules in a monolayer film, or "self-assembled monolayer" (SAM). The self-assembled monolayer may be selected from the group of aliphatic thiols and silanes.

The term "silane surface" as used herein refers to a silane used as a self-assembled monolayer attached to the surface of a medical device.

The term "transfection" as used herein refers to any method which is useful for introducing a nucleic acid molecule in an organism or cell. For example methods for transfection are calcium phosphate method, electroporation, lipofection, microinjection, particle gun, gene gun, *Agrobacterium tumefaciens-*mediated transfection, antibody-mediated transfection and combinations of these methods.

The present invention relates to a medical device comprising an antimicrobial peptide. Medical devices may be implants, instruments, sutures, carriers, dressings, viscoelastica and replacement body parts. The implant may be a metallic implant. Metallic implants include, but are not limited to, titanium, tantalum, cobalt base alloys and stainless steel implants. In the most preferred embodiment the implant is of titanium. The implants may be orthopedic and/or dental implants. Most preferably the implant is an orthopedic implant. Orthopedic implants may be for example plates, in particular bone plates, screws, in particular bone screws, pins, wires, femoral heads, nails, in particular hip nails, intramedullary interlocking nails and/or Kuntscher cloverleaf nails. The instrument may be a surgical instrument. Surgical instruments include, but are not limited to, instruments for bone screws, for bone plates, for pins, for wires, for femoral heads, for hip nails, for knee surgery, for skull surgery, retractors, elevators, hooks and levers, in particular Bennett's, hook for skin with three prong, skin hook-2 prong, cobs elevator, dental elevator L/R and cryer elevator; bone cutting instruments, forceps, chisels, osteotomes, gouges and curpets. The sutures may be, but are not limited to surgical sutures, which may be absorbable or non-absorbable. Implants may also be heart valves, ligaments, sinews, vasculatures, pericardium, temporary filling material of the skin matrix and replacement vitreous body. The medical device may be of, but is not limited to, gold, silver, stainless steel, titanium, gelatine, agarose, collagen, polylactide, hyaluronic acid and alginic acid.

The antimicrobial peptide according to the invention inhibits and/or kills pathogenic micro-organisms, for example bacteria, viruses, fungi, yeasts, mycoplasma and protozoa. Preferably, the antimicrobial peptide may be a member of the RNAse A super family, defensin, cathelicidin, granulysin, histatin, psoriasine, dermicidine or hepzidine. Members of the RNAse A super family are for example RNAse 1, RNAse 2, RNAse 3, RNAse 4, RNAse 5, RNAse 6, RNAse 7, RNAse 8 and RNAse 9. Defensins are for example alpha- and beta-defensins, like human beta-defensin-1, human beta-defensin-2, human beta-defensin-3, human beta-defensin-4, human alpha-defensin 1, human alpha-defensin-2, human alpha-defensin-3, human alpha-defensin-4, human alpha-defensin-5 and human alpha-defensin-6. Cathelicidin may be for example LL-37. Histatins are for example histatin 1, histatin 2, histatin 3, histatin 4, histatin 5, histatin 6 and histatin 7. Hepzidin may be for example hepzidin-20 or hepzidin-25. The antimicrobial peptide may be naturally occurring in insect, fish, plant or mammalian cells, preferably human cells. An advantage of the antimicrobial peptide is that the antimicrobial peptide exhibits no cytotoxic effects on humans and animals. Further, the antimicrobial peptide is active directly at the peri-implant site, thus, no systemic antimicrobial treatment is necessary. A further advantage is that the antimicrobial peptide can stimulate natural healing processes. Moreover, if the antimicrobial peptide is of human origin, then the antimicrobial activity of the human antimicrobial peptide as part of the coating of the medical device according to the invention takes place at its natural environment in the human body. Therefore, natural catabolism of the antimicrobial peptide is most likely.

Preferably, the antimicrobial peptide is a human antimicrobial peptide. In a preferred embodiment the antimicrobial peptide is a human 13-defensin. In the more preferred embodiment the antimicrobial peptide is human B-defensin-2, human B-defensin-3 or Ribonuclease 7.

The human B-defensin-2, human B-defensin-3 and Ribonuclease 7 have antimicrobial activity against several pathogenic micro-organisms.

In another preferred embodiment the medical device comprises two or more different types of antimicrobial peptides. More preferably the medical device comprises any combination of at least two different types of antimicrobial peptides selected for example from the group comprising human B-defensin-2, human ß-defensin-3 and Ribonuclease 7.

In a preferred embodiment human B-defensin-2 exhibits the amino acid sequence according to the SEQ ID NO: 1 or derivatives, fragments or homologues thereof. In another preferred embodiment human B-defensin-3 has the amino acid sequence according to the SEQ ID NO: 4 or derivatives, fragments or homologues thereof. In another preferred embodiment Ribonuclease 7 exhibits the amino acid sequence according to the SEQ ID NO: 7 or derivatives, fragments or homologues thereof.

The derivatives and fragments of the naturally occurring human B-defensin-2, human ß-defensin-3 and Ribonuclease 7 may be originated by mutations of the respective naturally occurring amino acid sequence, in particular by deletions, substitutions, insertions, additions or combinations thereof. The derivatives and fragments of the naturally occurring human B-defensin-2, human B-defensin-3 and Ribonuclease 7, respectively, have the antimicrobial activity of the human B-defensin-2, human B-defensin-3 and Ribonuclease 7, respectively, wherein the activity may be the same as the wildtype antimicrobial peptide, enhanced, reduced, but not completely lost.

The deletions introduced into the amino acid sequence of the naturally occurring human B-defensin-2, human ß-defensin-3 or Ribonuclease 7 according to SEQ ID NO: 1, 4 and 7, respectively may shorten the amino acid sequence, wherein the activity of the peptide may be the same as the wildtype peptide, enhanced, reduced, but not completely lost. The deletions may refer to one or several amino acids. If several amino acids are deleted, the deleted amino acids may follow directly consecutive. Further individual deleted amino acids or regions with several deleted amino acids may be separated from each other. Therefore in the naturally occurring amino acid sequence of human B-defensin-2, human B-defensin-3 and Ribonuclease 7, respectively, according to SEQ ID NO: 1, 4 and 7, respectively, one or several deletions may be introduced.

The substitutions introduced into the amino acid sequence of the naturally occurring human B-defensin-2, human ß-defensin-3 or Ribonuclease 7 respectively, according to SEQ ID NO: 1, 4 or 7, respectively, may alter the amino acid sequence, wherein the activity of the peptide may be the same as the wildtype peptide, enhanced, reduced, but not completely lost. The substitutions may refer to one or several amino acids. If several amino acids are substituted, the substituted amino acids may follow directly consecutive. Further individual substituted amino acids or regions with several substituted amino acids may be separated from each other. Therefore in the naturally occurring amino acid sequence of human B-defensin-2, human B-defensin-3 and Ribonuclease 7, respectively, according to SEQ ID NO: 1, 4 and 7, respectively, one or several substitutions may be introduced. For example the substituted amino acid sequence of Ribonuclease 7 is according to SEQ ID NO: 10.

The additions introduced into the amino acid sequence of the naturally occurring human B-defensin-2, human ß-defensin-3 or Ribonuclease 7, respectively, according to SEQ ID NO: 1, 4 or 7, respectively, may alter the amino acid sequence, wherein the activity of the peptide may be the same as the wildtype peptide, enhanced, reduced, but not completely lost. The additions may refer to one or several amino acids. If several amino acids are added, the added amino acids may follow directly consecutive. Further individual added amino acids or regions with several added amino acids may be separated from each other. Therefore in the naturally occurring amino acid sequence of human B-defensin-2, human B-defensin-3 and Ribonuclease 7, respectively, according to SEQ ID NO: 1, 4 and 7, respectively, one or several additions may be introduced.

Further N- or C-terminal fusion of a protein or peptide tag may be adjusted to immobilize the peptide on surfaces. The N- or C-terminal fusion of a protein or peptide tag may be for example His-tag, Strep-tag, Avi-tag, JS-tag, chemical biotinylation, PEGylation. Further the N- or C-terminal peptide tags may comprise Myc-tags or GST-tags.

All of the fragments, derivatives and homologues of the human B-defensin-2, human B-defensin-3 or Ribonuclease 7, respectively, according to the invention exhibit an antimicrobial activity that is also exhibited by the naturally occurring human ß-defensin-2, human B-defensin-3 or Ribonuclease 7, respectively. Furthermore, the above described mutations exhibit positive effects that are beneficial for a commercial use of the medical advice of the invention. Such positive effects may involve an enhanced protease stability, thermal stability or stability against chemical denaturing agents of the peptides immobilized on the medical device surface. The positive effect may also be expressed by an enhanced activity of the peptides.

In one embodiment of the invention the antimicrobial peptide as part of the coating of the medical device may be obtained by isolation of the peptide from the natural source. For example the human ß-defensin-2 and human B-defensin-3 naturally occur in human epithelial cells of the skin and the respiratory, urogenital and gastrointestinal tract. Ribonuclease 7 naturally occurs for example in human epithelial tissues including skin, respiratory tract, genitourinary tract and gut. Preferably, RNAse 7 naturally occurs in human keratinocytes.

In another preferred embodiment the antimicrobial peptide as part of the coating of the medical device is a recombinant antimicrobial peptide. In a more preferred embodiment the human B-defensin-2, human ß-defensin-3 and/or Ribonuclease 7 as part of the coating of the medical device is a recombinant human B-defensin-2, human B-defensin-3 and Ribonuclease 7, respectively.

The recombinant antimicrobial peptide as part of the coating of the medical device according to the present invention may be obtained by expression of the peptide in any other organism or cell culture than in the natural source, cultivation and raise, respectively of the organism or cell culture and subsequent isolation of the peptide from the organism, cell or the supernatant of the cell culture. Other organism includes, but is not limited to plants, bacteria, yeast and fungi. The cell culture includes, but is not limited to human, mammalian, insect and plant cell culture. Therefore, the cell or organism is transfected with the nucleic acid molecule encoding the respective antimicrobial peptide with all required transcription regulation elements. The methods used for transfection are for example calcium phosphate method, electroporation, lipofection, microinjection, particle gun, gene gun, *Agrobacterium tumefaciens-*mediated transfection, antibody-mediated transfection and combinations of these methods. The nucleic acid molecules encoding the antimicrobial peptides may comprise a precursor sequence at the 5' or 3' part of the nucleic acid molecule. The precursor sequence may be a signal sequence determining the localisation of the peptide in a cell or organism. The precursor molecule may be removed from the peptide during posttranslational modifications. It is known by the person skilled in the art that a start codon at the 3' part of the nucleic acid molecule is necessary to express the nucleic acid in a cell or organism.

In a preferred embodiment human β-defensin-2 is encoded by the nucleic acid according to SEQ ID NO: 2. In another preferred embodiment human β-defensin-3 is encoded by the nucleic acid according to SEQ ID NO: 5. In another preferred embodiment Ribonuclease 7 is encoded by the nucleic acid according to SEQ ID NO: 8.

In one aspect of the invention the antimicrobial peptide as part of the medical device according to the invention is encoded by a nucleic acid sequence with optimized codon usage pattern. Optimized codon usage pattern refers to the substitution of some of the codons coding for an amino acid of interest with such codons as to increase the expression level of the protein of interest in cells or tissues in different organisms. Various combinations of the codons to be substituted can be applied for achieving the increase in the expression level by those skilled in the art.

In a preferred embodiment human β-defensin-2, human β-defensin-3 and/or Ribonuclease 7 are encoded by a nucleic acid sequence with optimized codon usage pattern. For example human β-defensin-2 is encoded by the nucleic acid according to SEQ ID NO: 3, human β-defensin-3 is encoded by the nucleic acid according to SEQ ID NO: 6, Ribonuclease 7 is encoded by the nucleic acid according to SEQ ID NO: 9 and the Ribonuclease 7 derivative is encoded by the nucleic acid according to SEQ ID NO: 11.

In another preferred embodiment the antimicrobial peptide as part of the coating of the medical device is a synthetic antimicrobial peptide. In a more preferred embodiment the human B-defensin-2, human B-defensin-3 and/or Ribonuclease 7 as part of the coating of the medical device is a synthetic human B-defensin-2, human ß-defensin-3 and Ribonuclease 7, respectively.

The synthetic antimicrobial peptide as part of the coating of the medical device according to the present invention may be obtained by connecting one amino acid with another by forming a peptide bond. Methods for the production of synthetic peptides are for example the solid phase peptide synthesis (SPPS) and the liquid phase peptide synthesis (LPPS).

Preferably, the medical device according to the present invention further comprises a self-assembled monolayer. The self-assembled monolayer may be selected from the group of aliphatic thiols and silanes. Preferably self-assembled monolayers are silanes, in particular halogensilane, alkoxysilane, silazane and/or siloxane. Silanes may be branched or linear. Silanes may be silanes with at least one hydrogen substituted with an aliphatic hydrocarbon. The aliphatic hydrocarbon includes, but is not limited to chain-length of one up to and including 20 carbons. The aliphatic hydrocarbon may be branched or linear. Preferably the aliphatic hydrocarbon is linear. More preferably the self-assembled monolayer is an alkoxysilane. Alkoxysilanes include silanes which have at least one or more hydrogen substituted with an alkoxy group which includes alkyl residues with at least one carbon. In a more preferred embodiment the alkoxy group is a methoxy group. Especially preferred one or more of the aliphatic hydrocarbons include aliphatic hydrocarbons with terminal C=C, C=O, C-OH, COOH or C-NH₂ groups. In the most preferred embodiment the self-assembled monolayer may be a silane with at least one hydrogen is substituted with an alkoxy group, preferably a methoxy group and at least one hydrogen is substituted with an aliphatic hydrocarbon, wherein at least one of the aliphatic hydrocarbons exhibits a terminal C=C, C=O, C-OH, COOH or C-NH₂ group.

One aspect of the invention refers to a medical device having a silane surface comprising an antimicrobial peptide, wherein the antimicrobial peptide is attached to the silane surface. In a preferred embodiment the antimicrobial peptide is attached to silane via Van der Waals interactions, hydrophobic interactions and/or ionic interactions. Hydrophobic interactions may occur between the antimicrobial peptide and an aliphatic group of the self-assembled monolayer. Ionic interactions may occur between the antimicrobial peptide and a charged group of the self-assembled monolayer.

The self assembled monolayer according to the present invention may mediate the adherence to the medical device and the adherence of antimicrobial peptides. Preferably, the antimicrobial peptide attached to the self assembled monolayer may be human ß-defensin-2, human ß-defensin-3 or Ribonuclease 7. In a preferred embodiment the antimicrobial peptide may be attached to the self-assembled monolayer, preferably silane via a covalent bond. In another preferred embodiment the antimicrobial peptide may be attached to the self assembled monolayer, preferably silane via a terminal C=C, C=O, C-OH, COOH or C-NH₂ group. More preferably, the self-assembled monolayer, preferably silane may be attached to the medical device via an alkoxy group, even more preferably via a methoxy group.

Preferably, the medical device according to the present invention further comprises collagen. In a preferred embodiment collagen is attached to the self-assembled monolayer by simple coating, wherein the collagen is attached to the self-assembled monolayer by simple application, for example by dropping a collagen solution on the self-assembled monolayer and subsequently allows drying. If the collagen is attached to the self-assembled monolayer, preferably silane by simple coating, the attachment thereof may be occurred via Van der Waals interactions, hydrophobic interactions, ionic interactions and/or steric effects. In a preferred embodiment collagen is attached to the self-assembled monolayer, preferably silane by forming a covalent bond. The connection of the collagen and the self-assembled monolayer, preferably silane via a covalent bond may be obtained, for example by using glutaraldehyde and/or a covalently binding strategy with the NHS/EDC cross-linking system.

The present invention relates to a coated medical device according to the present invention to prevent infections. Infections include infections caused by bacteria, viruses and/or fungi. The infections by bacteria could be caused by gram-positive and/or gram-negative bacteria. Particularly infections by gram-negative bacteria are infections caused by *Aeromonas hydrophila, Acinetobacter baumannii, Acinetobacter calceoaceticus, Acinetobacter genosp. 3, Acinetobacter genosp. 10, Acinetobacter genosp. 11, Acinetobacter iwoffii, Acinetobacter junii, Acinetobacter johnsonii, Acinetobacter haemolyticus, Brevundimonas diminuta, Burkholderia cepacia Campylobacter jejuni, Citrobacter freundii, Enterobacter cloacae, Enterobacter aerogenes, Escherichia coli, Heliobacter pylori, Klebsiella pneumoniae, Morganella morganiii, Proteus mirabilis, Proteus vulgaris, Pseudomonas aeruginosa, Pseudomonas fluorescens, Pseudomonas putida, Providencia rettgeri, Providencia stuartii, Salmonella typhimurium, Serratia marcescens, Stenotrophomonas maltophilia* and/or *Yersinia enterococcus.* Particularly infections by gram-positive bacteria are infections caused by *Corynebacterium amycolatum, Corynebacterium pseudodiphtheriticum, Enterococcus faecalis, Enterococcus faecium, Propionibacterium acnes, Staphylococcus aureus, Staphylococcus epidermidis, Streptococcus pyogenes and Streptococcus pneumoniae.* The infections by viruses could be caused by adenovirus, papilloma virus, human immunodifficiency virus and the human herpes simplex virus. The infections by fungi could be caused by *Aspergillus niger, Candida albicans, Candida glabrata, Candida parapsilos, Candida tropicalis, Cryptococcus neoformans, Issatchenkia orientalis,* and/or *Saccharomyces cerevisiae.*

One further aspect of the present invention relates to the use of the coated medical device of the present invention for reduction of pathogenic microorganism colonization, in particular colonization of bacteria, viruses and/or fungi. The colonizing bacteria could be gram-positive and/or gram-negative bacteria. Particularly colonizing gram-negative bacteria are *Aeromonas hydrophila, Acinetobacter baumannii, Acinetobacter calceoaceticus, Acinetobacter genosp. 3, Acinetobacter genosp. 10, Acinetobacter genosp. 11, Acinetobacter iwoffii, Acinetobacter junii, Acinetobacter johnsonii, Acinetobacter haemolyticus, Brevundimonas diminuta, Burkholderia cepacia Campylobacter jejuni, Citrobacter freundii, Enterobacter cloacae, Enterobacter aerogenes, Escherichia coli, Heliobacter pylori, Klebsiella pneumoniae, Morganella morganiii, Proteus mirabilis, Proteus vulgaris, Pseudomonas aeruginosa, Pseudomonas fluorescens, Pseudomonas putida, Providencia rettgeri, Providencia stuartii, Salmonella typhimurium, Serratia marcescens, Stenotrophomonas maltophilia* and/or *Yersinia enterococcus.* Particularly colonizing gram-positive bacteria are *Corynebacterium amycolatum, Corynebacterium pseudodiphtheriticum, Enterococcus faecalis, Enterococcus faecium, Propionibacterium acnes, Staphylococcus aureus, Staphylococcus epidermidis, Streptococcus pyogenes and Streptococcus pneumoniae.* The colonizing fungi could be *Aspergillus niger, Candida albicans, Candida glabrata, Candida parapsilos, Candida tropicalis, Cryptococcus neoformans, Issatchenkia orientalis,* and/or *Saccharomyces cerevisiae.* The colonizing viruses could be in particular adenovirus, papilloma virus, human immunodifficiency virus and the human herpes simplex virus.

The following examples are provided merely by way of explanation and in no sense restrict the scope of invention.

### Example 1: Manufacture of coated titanium pins

Custom designed oxidized titanium pins were used in this study. Round-shaped pins of a size of 1 mm in height and 5 mm in diameter were made from titanium grade 4 according to ISO 5832-2 with surface roughness of 2-4 um.

### Cleaning and hydrophilisation of the Ti02 surface

Prior to surface modification, the pins were ultrasonicated (Sonorex Super 10P, Bandelin) for 10 min in 5 M KOH, for 10 min in 69% (v/v) HNO₃ and finally for 15 min in a 2:1 H₂SO₄/H₂O₂ mixture at room temperature. TiO₂ pins were washed out six times for 15 minutes in 15 ml distilled water. After that the pins were hydrophilised by incubating them for 1 h at 65°C in an oxidisation solution (NH₄OH/H₂O₂,/distilled water in a ratio of 1:1:1) and stored up to 16 h at 4°C in 70% (v/v) Ethanol.

### Functionalization of the titanium surface

Prior to coating of recombinant hβD2 on the titanium pins, the surfaces of the pins needed to be functionalised. This means that four different self-assembled monolayers (SAMI-4) were produced by direct silanisation of the hydrophilised pin surfaces. This means that four different self-assembled monolayers (SAMI-4) (Sigma- Aldrich Cat.: 28,177-8 and Cat.: 446955; Fluka Cat.: 52360 and Cat.: 68215) were produced by direct silanization of the hydrophilized pin surfaces. For silanisation the titanium pins were incubated either in 250uL of a 10% (v/v) solution of hexadecyltrimethoxysilane (SAMI) or dimethoxymethyloctylsilane (SAM2) or allyltrimethoxysilane (SAM3) or 3-aminopropyl-trimethoxysilane (SAM4) in toluol for 24 h at room temperature. Then the pins were extensively rinsed with toluol and dried by room temperature. Additional modification on silanized titanium surface were generated by oxidation of the CH=CH2 endgroups from allyltrimethoxysilane by incubation with 5% KMnO₄ acid aqueous solution and finally washed with distilled water.

### Collagen binding to the functionalised titanium surface (SAM 4)

To stably bind a collagen onto the functionalised titanium oxide surface, the protein fibrils were cross-linked with and the 3-aminopropyl-trimethoxysilane (SAM4) layer, respectively, in two different ways.

In the first case, SAM4 titanium pins, respectively, were pre-incubated in 200 mM 2-[N-morpholino]ethane sulfonic acid (MES) pH 5.5 for 1 h at room temperature. Twenty five µL of collagen solution from calf skin type I (0.1%, Sigma-Aldrich C8919) were pipetted on both surface sides of the pins and dried at room temperature. Cross-linking with N-hydroxysuccinimide (NHS) and N-(3-dimethylaminopropyl)-N'ethylcarbodiimide hydrochloride (EDC) was performed by incubating the pins in a solution of 10 mM NHS; 30 mM EDC in 200 mM MES pH5.5 for 6 h at room temperature (SAM 4::Co1-NHS).

Secondly, the dried collagen fibrils on the SAM4 titanium pin surface were cross-linked by incubating the pins in a 25 µL glutaraldehyde solution (25%,) for 1 h at room temperature. Subsequently the pins were rinsed in a 0.1%(w/v) bovine serum albumin (BSA) and 200 mM PBS solution for 20 min followed by an incubation in 100 mM Na₂HP0₄ for 1 h and finally washed in distilled water, dried in an exsiccator and stored at 4°C (SAM4::Co1-Glu).

The successful binding of collagen was monitored by a collagen-specific stain after treatment with 0.1% (w/v) DirectRed 80 (Fluka, Germany) in 0.5%(v/v) acetic acid. Samples were three times washed in 0.5M acetic acid. All chemicals were purchased from Sigma-Aldrich if not described otherwise.

### Coating of Human B-defensin-2 (hBD2) to the functionalised titanium surfaces

Five different groups of functionalised titanium pins were produced and investigated in this study: three silanised (SAM1, SAM2, SAM3) and two SAM4::Collagen (SAM4::Co1-NHS, SAM4::Col-Glu) titanium surfaces. Coating of this peptide was performed by directly adding 10 µl of a 1 mg/ml hβD2 or a 8 mg/ml solution (in 0.01% acetic acid) onto the functionalised titanium surfaces. Finally the pins were dried in an exsiccator and stored at 4°C prior to use. In this way, two sets of SAM1, SAM2 and SAM3 layers were generated by coating either 10 ug or 80 ug of hβD2 on each surface. The SAM4::collagen (SAM4::Col-NHS and SAM4::Col-Glu) functionalised titanium surfaces were coated with 80 ug of hβD2.

### Example 2: Antimicrobial activity of human B-defensin-2 coated to three SAM modified titanium pins against E. coli

In a first experiment 10 µg of hβD2 was coated to three different SAM Hexadecyltrimethoxysilane (SAM 1), Dimethoxymethyloctylsilane (SAM 2), and oxidized Allyltrimethoxysilane (SAM 3) of the modified titanium pins. The anti-bacterial activity of the hBD2-coated pins was tested by a micro-dilution assay. Therefore, *Escherichia coli* strain DB3.1 (Invitrogen, Cat. No. 11782-018) was used. E. *coli* was streaked from a glycerol stock onto a Brain Heart Infusion (BHI) agar plate (3.7% (w/v) BHI Bouillon, 1.5% (w/v) Agar), grown overnight at 37°C and subsequently used to inoculate 40 ml of 3.7%(w/v) BHI Bouillon without any antibiotics in 100 ml flasks. Fresh cells were harvested by centrifugation at 16000 rpm for 10 min and washed twice with 10 mM sodium phosphate buffer, pH 7.2. The optical density was adjusted to 10⁴ cells/ml. For anti-bacterial testing, a single pin coated with hβD2 was incubated in one well of a microtiter plate containing 100 µl of the bacterial suspension.

Functionalised titanium pins without hβD2 served as negative controls (SAM1-n, SAM2-n, SAM3-n, SAM4::Col-NHS-n, SAM4::Col-Glu-n). In addition, 5 independent control reactions consisting of hβD2 in a final amount of 0 (NC), 0.02 (PC1), 0.2 (PC2), 2 (PC3) and 10 (PC4) µg were examined in parallel. To determine the specificity of the anti-bacterial activity of hβD2, 10 µl (10 ug/ml in PBS) of polyclonal monospecific anti-hβD2 antibodies were co-incubated with 10 ug of hβD2 and bacterial cells (Table I, PC4/ab).

The microtiter plates were incubated for 2 h at 37°C. The kinetic studies were performed by replacing the bacterial suspension, after 2 h of cultivation with a freshly pre-cultured E. *coli* solution, which contained the same bacterial concentration, and cultivating for another 2 h and so on. Colony forming units (CFU) were determined after plating 100 µl of a 1:100 dilution of the bacterial suspension on petri dishes with BHI medium and incubating them overnight at 37°C. For every group of the functionalized titanium pins and the controls, 5 independent anti-bacterial assays were performed. A control (NC) of bacterial growth was examined for every independent assay. The number of CFU on the control (consisting of a single petri dish) was set to 0% bactericidal activity. The reduction in the CFU resulting from the anti-bacterial activity of hβD2 was related to the absolute CFU of the control within every independent assay. This value, given as a percentage of the bacterial "killing" activity, was used to describe the antibacterial activity. This kind of normalisation of the antibacterial data was performed for every experimental group.

In a parallel reaction, the amount of hβD2 released into the BHI medium was determined by an enzyme-linked immunosorbent assay (ELISA). BHI medium was prepared as described above without bacterial cells. Titanium pins were incubated in the BHI medium for 2 h at 37°C corresponding to the anti-bacterial assay. The same set of controls was examined as described above. Microtiter plates were coated with 50 µL of polyclonal monospecific rabbit anti-hβD2 antibodies (HBD21-A Alpha Diagnostic Inc. ADI San Antonio Texas) 100 ng/ml in 0.05 M Na₂C0₃ pH 9.6 and were then blocked with 0.5% BSA in PBS for 2 h at 37°C. After each step, the wells were washed three times with PBS, pH 7.4, containing 0.1% (v/v) Tween 20. Fifty µl of the sample, consisting of BHI medium that either contained the titanium pins or the control reactions, diluted in PBS/0.1% (v/v) Tween 20, was added in duplicate and incubated for 60 min at 37°C. After washing, 50 µL of the rabbit biotinylated anti- hβD2 antibody (100 ng/ml) was added followed by incubation for 60 min. After washing, horseradish peroxidase (HRP)-conjugated streptavidin was added and incubated for 45 min at 37°C. Finally the reaction was visualized by adding 50 µl tetramethylbenzidine (TMB) substrate for 10-20 min. The reaction was stopped with 0.5 M H₂S0₄ and the absorbance was determined at 450 nm using an ELISA plate reader. As a reference for quantification, a standard protein curve was established by a serial dilution of hβD2 (45 pg/ml to 1 µg/ml).

The SAM surfaces alone did not exhibit any anti-microbial activity prior to coating with hβD2 (Table I, SAM1-n, SAM2-n, SAM-3n). In contrast, functionalised titanium pins coated with hβD2 exhibited an antibacterial activity of more than 90% killing (Table I, SAM1-3). The biological activity of the coated titanium pins corresponded to the positive hβD2 control (PC4) with the highest concentration (10 µg) and demonstrated successful coating of the pins with hβD2. Furthermore, the ELISA data presented in Table I confirmed (i) that coating with hβD2 of the functionalised pins was nearly 100%, (ii) that biologically active hβD2 was eluted from the pins, and (iii) that the discrepancy between the amount of hβD2 applied for coating and the amount eluted from the pins indicate a storage function of the SAM layers for hβD2.

The aliphatic SAM-layers (SAM1 and 2) and also the negatively charged SAM3-layer could store and release sufficient hβD2 for anti-microbial activity. However, differences between the three functionalised surfaces could be observed. Both hydrophobic surfaces (SAM1 and 2) exhibited a higher capacity for storage and peptide release compared to the SAM3 pins. The slow release of hβD2 led to a better anti-microbial efficacy. The reason therefore could be that SAM3, which did not exhibit the same binding capacity for hβD2 compared to the other SAM surfaces, could offer negatively charged groups for the interaction with hβD2.

**Table I:**

| sample group | rHußD2 controls | | | | | |
|---|---|---|---|---|---|---|
| sample | NC | PC 1 | PC2 | PC3 | PC4 | PC4/ab |
| amount of applied rHußD2 [µg] | 0 | 0.02 | 0.20 | 2.00 | 10.00 | 10.00 |
| | | | | | | |
| amount of determined rHuBD2 [ug] by ELISA | o | 0.02 ±0.01 | 0.18 ±0.01 | 1.70 ±0.14 | 9.01 ±0.82 | 8.89 ±0.72 |
| | | | | | | |
| antmicrobial activity, bacterial killing [%] | 0 | 14.80 ±2.78 | 32.38 ±2.93 | 92.80 ±3.91 | 100 | 0 |
| | | | | | | |

| sample group | Titanium: SAM1 | | Titanium:SAM2 | | Titanium:SAM3 | |
|---|---|---|---|---|---|---|
| sample | SAM1-n | SAM1 | SAM2-n | SAM2 | SAM3-n | SAM3 |
| amount of applied rHuBD2 [ug] | 0 | 10.00 | 0 | 10.00 | 0 | 10.00 |
| | | | | | | |
| amount of determined rHuBD2 [µg] by ELISA | 0 | 6.77 ±1.72 | o | 8.05 ±2.14 | 0 | 5.35 ±1.32 |
| | | | | | | |
| antmicrobial activity, bacterial killing [%] | 0 | 93.20 ±3.78 | 0 | 95.63 ±4.39 | 0 | 89.83 ±3.98 |

### Example 3: Release kinetics of hßD2 adsorbed by three SAM modified titanium pins against E. coli

To examine the release behaviour of hβD2 that is adsorbed by different SAM modified titanium pins a time kinetic experiment was performed.

The experiment was performed analogous to Example 2. For this experiment 80 µg of h13D2 was adsorbed to three different SAMs Hexadecyltrimethoxysilane (SAM1), Dimethoxymethyloctylsilane (SAM2), and oxidized Allyltrimethoxysilane (SAM3) of the modified titanium pins.

A high anti-microbial activity with a killing rate of 100% was observed for all SAMs after 2 h of incubation in the anti-bacterial assay. ELISA quantification revealed that at this time point, about 74-78 µg hßD2 was eluted from the different titanium pins into the medium (Table II). The time- dependent cultivation of the titanium pins was performed by incubating the same pin after the first two hour cultivation in a freshly prepared E. *coli* culture containing the same amount of bacteria for another two hours and so on. It could be shown that after the second cultivation step (4 h) the anti-microbial activity still reached killing rates of 52% (corresponding to 0.41 µg hBD2 in the medium, Table II) to 69% (corresponding to 1.29 µg hBD2 in the medium, Table II). At this time point, a statistically significant stronger killing activity of hβD2 of the SAM2 pins compared to the other functionalised surfaces could be observed. After 6 h, the killing rate for SAM2 was still 60% (corresponding to 0.65 ug hD2 in the medium), that for SAM1 was about 29% (corresponding to 0.08 ug hβD2 in the medium) and it dropped significantly for SAM3, below 5%. Only SAM2 exhibited a significant reduction of bacterial colonisation of about 5 % after 8 hours of cultivation. Almost no antimicrobial activity could be observed with the other two SAMs, SAM1 and SAM3. These findings indicated that different functionalised titanium pins revealed a different elution profile in a defined time span.

**Table II: hBD2 released amount in the medium determined by ELISA**

| | SAM1 | SAM2 | SAM3 | SAM4:Col-Glu | SAM4:Col-NHS |
|---|---|---|---|---|---|
| 2h | 76.18 ± 1.72 | 74.28 ± 1.11 | 78.85 ± 1.12 | 17.20 ± 4.30 | 14.31 ± 1.72 |
| 4h | | | | 6.62 ± 2.54 | 0.46 ± 0.22 |
| | 0.61 ± 0.24 | 1.29 ± 0.38 | 0.41 ± 0.16 | | |
| 6h | 0.08 ± 0.03 | 0.65 ± 0.08 | 0 | 0 | 0 |
| 8h | 0 | 0 | n.d. | n.d. | n.d. |

It could be shown that coating with 80 µg of hβD2 revealed continuous release of hβD2 for several hours. Hexadecyltrimethoxysilane (SAM1) and dimethoxymethyloctylsilane (SAM2) differ in the length of their exposed aliphatic chains. The aliphatic chain of dimethoxymethyloctylsilane (SAM2) is shorter and might exhibit a better hβD2 delivery system indicated by an anti-microbial activity of 60% killing after three cultivation steps (6 h). The other hydrophobic layer (SAM1) exhibited a killing rate of 29% only.

Surprisingly, the functionalised SAM3-surface exhibited the fastest decrease of activity over time. After six hours, almost no killing of E. *coli* was observed and no hβD2 was detected by ELISA. A possible explanation for this behaviour is the low binding capacity of SAM3 so that most of hβD2 was already delivered after 2 h of cultivation. Bacterial contamination of implants and hence infections, occur often immediately after implant integration. In consequence, the silane functionalised titanium surfaces enable the immediate release of huge amounts of hβD2 in a short time in vitro which may prevent peri-implant infections in clinical use.

### Example 4: Antimicrobial activity of hßD2 coated on collagen modified titanium pins against E. coli

Since it is known that collagen has a positive impact on wound healing, functionalised titanium pins with collagen were investigated. Two different cross-linking strategies for collagen on SAM3 and SAM4 were applied as described in example 1: (i) a covalently binding strategy with the NHS/EDC cross-linking system, and (ii) the use of glutaraldehyde. Successful binding of collagen was monitored by collagen-specific DirectRed staining (as described in example 1). To control the coating of collagen of the modified titanium pin SAM4 (3-Aminopropyl-trimethoxysilane) has been used because it exhibited antimicrobial activity. Additionally 80 ug hBD2 was adsorbed to collagen functionalized SAM3 and SAM4 modified titanium pins, respectively. For monitoring the antibacterial activity of the bio-coated pins the experiment was performed analogous to example 3.

Functionalised collagen titanium pins without hßD2 did not show any anti-bacterial activity (data not shown). After two hours of incubation, SAM4 modified titanium pins of both cross-linking strategies generated killing rates of 100%. However, ELISA quantification of these pins revealed a release of only 14-17 µg of hßD2 into the medium (Table II). After 4 h of cultivation, the killing rate dropped to 53% (corresponding to 0.46 pg of hBD2 in the medium) for the NHS/EDC-system whereas collagen pins treated with glutaraldehyde exhibited a killing rate of almost 92% (corresponding to 6.6 ug of hBD2 in the medium). After 6 h, the anti-microbial activity of both systems dropped below 10% of the killing rate and no hßD2 was detectable in the medium by ELISA.

The direct comparison of collagen-treated surfaces and pure silane monolayers revealed that less hβD2 was eluted into the medium from collagen pins (Table II). This effect might be associated with a strong and irreversible binding of hβD2 onto the collagen matrix. Within the first two hours of the anti-bacterial assay of the collagen titanium pins, only 14-17 ug hßD2 was detected by ELISA which led to a complete killing of the bacteria. However, after the second cultivation step (4 h), a statistically significant difference was observed between hβD2 release from the glutaraldehyde cross-linked collagen surface (SAM4::Co1-Glu) and the other collagen surface (SAM4::Col-NHS). SAM4::Col-Glu delivered about 6.6 ug hßD2 and caused 92% of bacterial killing. In contrast, SAM4::Col-NHS released only 0.46 ug hBD2 with an antibacterial activity of 53%.

After two hours of incubation, SAM3 modified titanium pins of both cross-linking strategies generated killing rates of 96%. For the SAM3 modified titanium pins no ELISA quantification was performed. After 4 h of cultivation, the killing rate dropped to 84% for the NHS/EDC-system whereas collagen pins treated with glutaraldehyde exhibited a killing rate of almost 74%. After 6 h, the anti-microbial activity of both systems dropped to 0% of the killing rate.

It was not possible to determine the bioactive hBD2 remaining in the collagen matrix. However, it can be speculated that as a consequence of gradual collagen catabolism in the surrounding tissue in vivo, bioactive hβD2 might be slowly released. On the basis of this hypothesis, a peptide delivery system that will yield defined anti-microbial dosages in vivo could be developed in the future by using different collagen binding systems.

### Example 5: Preparation of different hßD2 coated biopolymers

### Material preparation:

### - Polylactide matrices:

Round pads crocheted of a polylactide thread, in the three variants uncoated, coated with collagen and coated with collagen and chondroitin sulphate were cut into samples with a diameter of 5 mm. The surface of these samples were dropped with 25 ug hBD2 in 10 µl 0.01 % acetic acid and were afterwards dried in an exsiccator for 15 min at room temperature.

### - Collagen scaffolds:

Collagen scaffolds with porous, spongy structure were produced by punching out of samples with a diameter of 6 mm. Afterwards the samples were washed to remove bio-active substances remaining in the matrix of the scaffolds during the production procedure. The washing comprises three incubations of the matrix in 40 ml 20 % ethanol, 10 mM natrium phosphate buffer and distilled water of one hour each at room temperature under strong movement. Subsequently, the scaffolds were dried in an exsiccator with heat applied by a heated metal plate for 1h on a Teflon block, after drying different amounts of hBD2 in 10 µl 0.01 % acetic acid were dropped on the scaffolds which were then dried again. A sample had a diameter of 4 mm (+/- 10%) and a mass of 1 mg (+/- 10 %) bovine collagen type I.

### - Biopolymer films:

The biopolymers hyaluronic acid, alginic acid and agarose were diluted each in distilled water to obtain a 1% solution. The alginic acid solution and the agarose solution were additionally boiled. On a heat plate the drops of 50 µl solution were dried to films with a diameter of about 5 mm on a Teflon block. Samples of gelatin leaves were produced by punching out samples with a diameter of 5 mm. After mixing the samples with 10 ug hBD2 in 10 µl 0.01% acetic acid the samples were dried on a Teflon block in an exsiccator for 15 min at room temperature.

### Microdilution assay:

In the microdilution assay the microorganisms were exposed to a potential bio-active substance in liquid surrounding, thus free to move. This potential bio-active substance was either homogenously dispersed in the liquid surrounding by active mixing at the beginning of the tests or could spread out of the sample during incubation time. The bioactivity was measured in percentage killing, wherein the number of colony forming units (CFU) in a control without test substance was set to 0% killing and a reduction in the cell number to zero was set to 100% killing.

Therefore, *Escherichia coli* strain DB3.1 (Invitrogen, Cat. No. 11782-018) was used. *E. coli* was streaked from a glycerol stock onto a Brain Heart Infusion (BHI) agar plate (3.7% (w/v) BHI Bouillon, 1.5% (w/v) Agar) and was grown over night at 37°C. Subsequently one colony was used to inoculate 10 ml of 3.7% (w/v) BHI Bouillon without any antibiotics in a culture tube which was then incubated shaking (120 rpm) over night at 37°C. 100 µl of this preculture was used to inoculate again 10 ml of 3.7% (w/v) BHI Bouillon without any antibiotics in a fresh culture tube which was then incubated shaking (125 rpm) for 2.5 h at 37°C. Fresh cells were harvested by centrifugation at 1600 rpm for 10 min, the supernatant was removed and the cell pellet was resuspended in 5 ml 10 mM sodium phosphate buffer. This procedure was repeated twice. The optical density was adjusted to OD600 of 0.500 with 10 mM sodium phosphate buffer. 100 µl of the bacterial suspension was pipetted in one well of a microtiter plate without optimized surface. 10 µl test substance or test sample was added. In case of a test substance, the mixture was homogenized by absorbing the mixture several times with a pipette. The microtiter plate is then closed with parafilm and incubated at 37°C for 2 h. After incubation the substances in the wells were mixed by a pipette. For the determination of the remaining number of colony forming units the bacterial suspension of the wells were diluted 1:100 with 10 mM sodium phosphate buffer and 100 µL of this dilution was streaked onto a Brain Heart Infusion (BHI) agar plate (3.7% (w/v) BHI Bouillon, 1.5% (w/v) Agar) and grown over night at 37°C. For evaluation, the grown colonies were counted.

### Agar diffusion assay:

In the agar diffusion assay the microorganisms were exposed immovable to the potential bio-active substance by inclusion in an agar containing medium. The use of a nutrient limited minimal medium led first to a limitation of the development of the bacteria. The test substances were dependent on the application form pipetted into the punched recess in the agar or placed with a test sample into the punched recess in the agar and spread out during incubation time by diffusion into the agar. After incubation the nutrient limitation of the bacteria was revoked. The determination of the bioactivity was performed by measuring the diameter of the raised zone of inhibition surrounding the recess.

Therefore, *Escherichia coli* strain DB3.1 (Invitrogen, Cat. No. 11782-018) was used. *E. coli* was streaked from a glycerol stock onto a Brain Heart Infusion (BHI) agar plate (3.7% (w/v) BHI Bouillon, 1.5% (w/v) Agar) and was grown overnight at 37°C. Subsequently one colony was used to inoculate 8 ml of TSB medium (Caso Bouillon 3% (w/v)) without any antibiotics in a culture tube which was then incubated shaking (120 rpm) over night at 37 °C. 50 µl of this pre-culture was used to inoculate again 8 ml of TSB medium (Caso Bouillon 3% (w/v)) without any antibiotics in a fresh culture tube which was then incubated shaking (125 rpm) for 3.5 h at 37°C. The OD₆₀₀ of the bacterial suspension was then 0.500.

The underlay medium (11% (v/v) 0.1 M sodium phosphate buffer pH 7.2, 1% (v/v) TSB-medium, 0.02% (v/v) Tween, 1 % (w/v) agarose in distilled water, pH 7.2), a minimal medium, was liquefied using micro waves and cooled to about 47°C. 10 ml underlay medium with 500 µl bacterial suspension were mixed in a 50 ml falcon tube by slight swirling and then was poured into a petri dish. The cooling time was first 15 min at room temperature and then 30 min at 4°C. Afterwards the recesses for test liquids (diameter 3 mm) and test materials (diameter 6 mm), respectively, were punched out from the underlay medium by using a punching-dye. 5 µl test liquid was pipetted per recess and the test materials were laid into the recess or were slid under the underlay medium, respectively. The lid of the plate was closed by parafilm and the plate was incubated over night at 37°C. The next day, the overlay medium (3.4% (w/v) casein peptone, 0.6% (w/v) soy flour peptone, 0.5% (w/v) di-potassium hydrogen phosphate, 1% (w/v) NaCl, 0.5% (w/v) glucose, 1% (w/v) agarose in distilled water) was liquefied using micro waves and cooled to about 47°C. In every Petri dish 10 ml overlay medium were poured on the underlay medium. After cooling for 5 minutes the plate was closed by the lid and incubated at 37°C for about 2-3 h. For evaluation, the diameter of the raised zones of inhibition surrounding the recesses was measured.

### Example 6: Antimicrobial activity of hßD2 coated on different biopolymers against E. coli

The potential of the chosen biopolymers to release bio-active hBD2 after coating/mixing them with hBD2, was determined by a screening in the diffusion assay (as described in example 5). The assay allows a correlation between the released amounts of bio-active molecules and the resulting zone of inhibition surrounding the releasing source. A comparison with the effect of directly applied, defined amounts of hBD2 allows an evaluation of the release potential of the test materials.

In the assay films made of 1% hyaluronic acid and 1% alginic acid, gelatin leaves, gelatinized agarose drops of 1% agarose solution and films of the dried drops were used. Additionally three variants of a polylactide matrix (uncoated, coated with collagen, coated with collagen and chondroitin sulphate) and collagen scaffolds were tested.

The samples were prepared as described in example 5 and treated with 10 µg hBD2. For the comparison between the effect of directly applied hBD2 amounts and the effect of hBD2- coated samples a standard series for 0.1, 1, 5 and 10 µg hBD2 was performed. The control was formed by the 0.01% acetic acid. The control with 0.01 % acetic acid shows no formation of zones of inhibition, the standard series exhibits zones of inhibition with diameters from 6 mm in case of 0.1 ug hBD2, 9 mm in case of 1 ug hBD2, 12 mm in case of 5 ug hBD2 and 15 mm in case of 10 ug hßD2. The biopolymers without hBD2 did not lead to the formation of zones of inhibition. The zones of inhibition surrounding the biopolymers treated with hßD2 showed that a complete release of the used hBD2 amount of 10 ug exhibited only in case of gelatin leaves and agarose, in case of the latter both of gelatinized agarose drops and of drops dried to films. The diameter of the zone of inhibition surrounding the test materials corresponded to the zone of inhibition caused by direct application of 10 µg hBD2 in the assay. In contrast, hyaluronic acid and alginic acid treated with hBD2 did not exhibit the formation of a zone of inhibition. The hBD2 applied on these sample materials was completely retarded and thus no release of bio-active molecules occurred. The collagen scaffold caused significant zones of inhibition in the assay, but a complete release of hBD2 during the time frame of the assay did not occur. At an average, 40% of hBD2 was released by the collagen scaffold and 60 % was retarded. The hBD2 release of the polylactide matrix was about 70% in case of all three variants and thus was higher compared to the release of the collagen scaffold.

Since the sample materials of hyaluronic acid and alginic acid did not allow the release of hBD2 as a bio-active substance, these materials were not appropriate for further experiments. In contrast, agarose and gelatine exhibited a hBD2 release of 100%. The hßD2 molecules were retarded stronger by the collagen than by the gelatine. Only 40% of the applied hBD2 molecules were released in a bio-active form, the remaining 60% remained in the scaffold or were released as inactive molecules. If the hßD2 amounts remaining in the scaffold are active molecules, this represents a loss of biocidal effect in a medical application in the body, as soon as the collagen is degraded by proteases, a release of the remaining hBD2 molecules which will become effective, can be possible.

Polylactides have hydrophobic properties caused by methyl groups in the molecule. Thus, there can be hydrophobic interactions between the applied hßD2 molecules and the polylactide matrix. hßD2 seems to be attached to the matrix only in a loose way, because 70 % of the hBD2 molecules can be released and only 30% remain on the surface as loss and being inactivated. The polylactide matrices coated with collagen and condroitin sulphate achieve in the diffusion assay the same hßD2 release amount as the uncoated matrix, because the retarding effects of the collagen and the negatively charged chondroitin sulphate on the positively charged hBD2 molecules are relativized by the long incubation time of the samples in the assay.

### Example 7: Release kinetics of hßD2 adsorbed by different biopolymers against E. coli

The microdilution assay facilitates the examination of the hBD2 release kinetics of the biopolymers and thus allowed conclusions on what amounts of hBD2 in which time could be released. For comparing on evaluation of the assay the effect of directly applied hBD2 amounts with the effect of hBD2 amounts applied on biopolymers, the achieved percentage killing of bacteria was compared to the killing rate of the hBD2 standard series in the microdilution assays.

Since gelatin leaves and agarose exhibit a high swelling capability in aqueous environment, the sample materials strongly increased the viscosity of the bacterial suspension in the assay that it was not possible to remove them from the wells. Thus, the analysis of the assay was not possible and these sample materials were not used further in the examination of the hßD2 release kinetics with the microdilution assay.

The collagen scaffolds and polylactide matrices were examined in the microdilution assay regarding their hBD2 release kinetics. Since the incubation time of the samples in the bacterial suspension is limited, the samples were stored at -25°C after the test and until completion of a further assay. The thawed samples were then used in a new assay and were incubated again. This succession was repeated since the hBD2 release potential of the samples is depleted and no bioactivity is detectable.

### Polylactide matrix

The hBD2 release kinetics of the polylactide matrix in the three variants uncoated, coated with collagen and coated with collagen and chondroitin sulphate could be monitored in the microdilution assay, as described in example 5, in a time frame of 0 to 12 hours. The sample materials were treated each with 25 µg hßD2. Without hßD2 coating the matrices show no antimicrobial activity, they caused no inhibition of the development of the colony forming units in the assay (Data not shown).

The uncoated and coated with collagen variants showed in the first two hours of the assay a bacteria killing of 100%, what corresponds to a killing of 4 x 10⁴ colony forming units and a hßD2 release of at least 5 ug compared with the achieved killing rates of directly applied hßD2-solutions in the assay. The matrix coated with collagen and chondroitin sulphate caused in this time frame only a killing of 89%, what corresponds to a release of maximal 2 µg hBD2. In the process of the measurement times the uncoated matrix fastest lost bio-activity, the percentage killing decrease the most. After 4 hours the hßD2 release was about 1 ug, after 9 hours the hBD2 release was reduced to 0.2 ug.

The matrix coated with collagen showed the slowest loss of bioactivity. In consideration of the whole time frame, the collagen coated matrix provided the highest bioactivity of the three variants. The deviations between the three matrices regarding their effect as hBD2 depot were at a maximum of 19%. All three variants allowed a continuous hBD2 release for several hours, after 12 hours the bioactivity was nearly depleted. The killing rate was very low and corresponded to h13D2 release of less than 0.02 ug.

A polylactide matrix in three variants uncoated, coated with collagen and coated with collagen and chondroitin sulphate and treated with 25 ug hßD2 each showed in the microdilution assay a bio-active effect for 12 hours. In the first 2 hours of the assay the uncoated variant and the variant coated with collagen caused the complete killing of the bacteria in the assay. The exact amount of released hBD2 could not be determined, because a killing rate of 100% without fluctuations occurred from a release of at least 10 ug hßD2. Since after 4 hours only a release of a maximum of 2 ug occurred and the release strongly decreased further, it can be concluded that assuming the complete release of the original 25 µg hBD2 during the 12 hours of the assay, the largest portion of the bioactive molecules had to be already released in the first 2 hours.

A polylactide matrix coated with hBD2 is useful for medical applicability which requires a fast release of large amounts of bio-active molecules and afterwards manages with lower release amounts for several hours. In case of strong local bacterial contamination of the application site a rapid killing of the major part of the bacteria could be achieved and a killing of the rest of the contamination in the subsequent hours.

Since the additives collagen and chondroitin sulphate have wound healing promoting properties like support of haemostasis, formation of new tissue and vascularisation and the differences in the hBD2 release kinetics between the three matrix variants are rather low, the use of coated polylactide is more useful than the use of pure (uncoated) polylactide.

### Collagen scaffolds

The hBD2 release kinetics of the collagen scaffolds were monitored in the microdilution assay, as described in example 5, in a time frame of 0 to 20 hours. The scaffolds were treated with different amounts of hßD2 (2; 4; 30; 125 µg) to determine the influence of the hBD2 concentration on the hBD2 release kinetics. Without hBD2 coating the scaffolds exhibited no antimicrobial activity, they caused no inhibition of the development of the colony forming units in the assay (Data not shown).

A hBD2 amount of 2 ug on a scaffold caused killing rates of a maximum of 36 % ± 12 in the first 2 hours of the assay, what corresponded to a hBD2 release of about 0.2 ug. The release amount decreased in the further time course, after 13 hours the bacteria killing of only 18 % could be achieved, what corresponds to a hBD2 release of about 0.02 ug. Also after 17 hours the released amount of bioactive molecules remained on this level, the fluctuations of the values were about 10%. After 20 hours no hßD2 release could be detected.

The increase of the hBD2 amount to 4 ug allowed in the first 2 hours the killing of 89 % of the bacteria, what corresponded to a hßD2 release of about 2 ug. In the process of the first 5 hours the release amount decreased to 38%, thus only about 0.2 ug hßD2could be active in the assay. Until the 17^{th} hour the killing was further reduced to 25%, after 20 hours the bio-activity of the scaffolds was depleted. The fluctuations of the killing rates were lower than the fluctuations of the scaffolds coated with 2 µg hBD2.

A scaffold coated with 30 ug hBD2 achieved in the beginning a killing rate of 98 to 100 %, what corresponds to a hBD2 release of at least 5 µg. In the first 5 hours the killing rate decreased to 86%, what corresponded to a release of hBD2 of about 2 ug. After 13 hours the release was 0.2 µg and achieved a killing of 29%, after 17 hours the killing rate was 22% and after 20 hours this scaffold was inactive.

In case of a scaffold coated with 125 µg hBD2 compared to 30 ug hßD2, only an increase of the release amount was achieved after 5 hours from 86% to 93%. A significant improvement of the release or an extension of the release time did not occur, also in this case the hBD2 release was zero after 20 hours.

Taking into consideration the available hBD2 amounts on the collagen scaffolds and the achieved killing rates in the assays, it is obvious that all scaffolds retard a part of the hBD2 molecules and thus prevent that the complete potential of bio-activity could be exhausted. The hBD2 amount on a scaffold has to be selected higher than the amount being usually sufficient to achieve the desired effect. With the excess on hBD2 molecules the binding sites in the scaffolds are saturated and the remaining molecules could be released. The blocking effect occurred here from a hßD2 amount of 30 µg per scaffold. Although a part of the molecules remained in the scaffold, high release rates could be achieved for at least 5 hours. Regarding their medical applicability the collagen scaffolds coated with hBD2 are useful for applications requiring a continuous release of bio-active molecules for several hours. An application site could be dispensed from low local bacterial contamination and is protected against resettling in the following times.

### Blocking with amino acids

The achieved killing rates of especially low hßD2 amounts per scaffold showed that a large portion of bio-active molecules were not removed from the collagen and thus hBD2 losses occurred. For preventing this, it was tried to saturate the collagen scaffolds with other molecules before coating with hBD2 and to block binding mechanisms. Therefore the collagen scaffolds were impregnated with different amino acids and dried in the exsiccator before the scaffolds were coated with hßD2 and again dried. Alternatively, the hBD2-solution was mixed with the respective amino acid before the scaffolds were treated with them and dried. L-lysine, poly-L-lysine, L-glutamic acid and poly-L-glutamic acid were used. Afterwards these pre-treated scaffolds were used in a microdilution assay as described in example 5.

In the microdilution assay L-lysine exhibited a low self-activity and reduced the number of bacteria by 4%. Scaffolds impregnated with 20 µl of a 0.1 M L-lysine solution, dried and subsequently treated with 4 ug hBD2, only achieved a killing rate of 11%. In contrast, scaffolds without pre-treatment with an amino acid exhibited a killing of 86%. Poly- L- lysine itself caused a killing of the bacteria of 100%, and thus could not be used for the blocking of the scaffolds. L-glutamic acid, as well as L-lysine had a low self-activity of 4%. Scaffolds impregnated with 20 µl of a 0.1 M L-glutamic acid solution, dried and subsequently treated with 4 µg hßD2, only achieved a killing rate of 53%. Poly- L- glutamic acid exhibited no self-activity, but the killing rate of 4 ug hßD2 on the scaffolds treated with 60 ug of the poly amino acid was reduced to 23%.

The amino acids L-lysine, L-glutamic acid and poly-L-glutamic acid deteriorated the achievable killing rates significantly. The hBD2 molecules were either more effectively be retarded in the scaffold or prevented in their bio-active activity by these additives.

### Blocking with proteins

For the determination of the effect of protein additives in collagen scaffolds on the hBD2 release the scaffolds were subjected to a pre-treatment with the biopolymer gelatine and the globular proteins bovine serum albumin (BSA) and human serum albumin (HSA). After impregnation with the protein solutions and drying, the scaffolds were treated with 4 ug hBD2. These pre-treated scaffolds were then used in a microdilution assay as described in example 5.

None of the proteins showed self-activity in the microdilution assay (data not shown). Due to the pre-treatment of the scaffolds with 10 µl of a 0.1% gelatine solution the hßD2 release increased and allowed a killing of 94% compared to the scaffold without gelatine only achieving a killing rate of 86%. The scaffolds treated with 5 ug BSA achieved a killing rate of 98%, after a treatment with 5 ug HAS a killing of the bacteria of 100% was possible.

A pre-treatment of the scaffolds with the proteins gelatine, BSA and HAS exhibited an improvement of the hBD2 release rate. The binding sites of the collagen were blocked, bio-active molecules could be released and the achievable killing rates increased.

### Blocking with further substances

As further possible blocking substances chondroitin sulfate, sodium citrate and spermidine was used. Also here the collagen scaffolds were impregnated with the diluted substances, dried in the exsiccator, treated with hßD2 and dried again. These pre-treated scaffolds were used in a microdilution assay as described in example 5.

Chondroitin sulphate exhibited in the microdilution assay a self-activity of 23%. A collagen scaffold treated with 5 ug chondroitin sulphate and 4 ug hßD2 achieved a killing rate of 54%. Sodium citrate caused the killing of the bacteria of 100% in the assay and thus could not further used. Pure spermidine reduced the number of bacteria by 35%, in case of combination of 20 ug spermidine and 4 ug hßD2 on a scaffold the achieved killing was 19%.

Chondroitin sulphate and spermidine deteriorated the achievable killing rates. As well as in the blocking with amino acids the hßD2 molecules were either more effectively retarded in the scaffold or prevented in their bio-active activity by these additives.

### Example 8: of hßD2 against various bacteria and funghi

The anti-bacterial activity of hBD2 was tested by a micro-dilution assay. The respective bacteria and fungi strains given in Table III were used. Bacteria and fungi were streaked from a glycerol stock onto a Brain Heart Infusion (BHI) agar plate (3.7% w/v BHI Bouillon, 1.5% w/v Agar), grown overnight at 37°C and 30°C, respectively and subsequently used to inoculate 40 ml of 3.7% w/v BHI Bouillon without any antibiotics in 100 ml flasks. Fresh cells were harvested by centrifugation at 16000 rpm for 10 min and washed twice with 10 mM sodium phosphate buffer, pH 7.2. The optical density was adjusted to 10⁴ cells/ml. For anti-bacterial testing hβD2 was incubated in one well of a microtiter plate containing 100 µl of the bacterial suspension. Therefore, ten microlitres of hßD2 solution with a range of final concentrations tested from 0.0125 up to and including 100 mg/l was added to the bacterial suspension and incubated at 37°C and 30°C, respectively for 2 h before colony forming units were determined. Colony forming units (CFU) were determined after plating 100 µl of a 1:100 dilution of the bacterial suspension on petri dishes with BHI medium and incubating them overnight at 37°C. For every group of the hBD2 added samples and the controls, 5 independent anti-bacterial assays were performed. A control (NC) of bacterial growth was examined for every independent assay. The number of CFU on the control (consisting of a single petri dish) was set to 0% bactericidal activity. The reduction in the CFU resulting from the anti-bacterial activity of hβD2 was related to the absolute CFU of the control within every independent assay. This value, given as a percentage of the bacterial "killing" activity, was used to describe the antibacterial activity. This kind of normalisation of the antibacterial data was performed for every experimental group.

The antibacterial activity of hβD2 were given either as minimum bactericidal concentration (MBC), i.e. the minimum concentration of hBD2 in ug/ml which is required to kill 99.9 % of the bacteria or fungi or as lethal dose (LD90), i.e. the concentration of hBD2 in µg/ml which is lethal for 90 % of bacteria or fungi. The values of the MBC and LD90 for the respective strain are given in Table III.

**Table III**

| **Bacteria Strains** | **MBC = µg/ml** | **LD 90 = µg/ml** |
|---|---|---|
| | **99.9 % killing** | **90 % killing** |
| **gram positive bacteria** | | |
| | | |
| **Staphylococcus aureus ATCC 12600** | **100** | **25** |
| **Staphylococcus aureus ATCC 33593 (MRSA)** | **>100** | **100** |
| **Staphylococcus aureus ATCC 43300 (MRSA)** | **>100** | **100** |
| **Staphylococcus aureus (MRSA 344) (wild type)** | **100** | **100** |
| **Staphylococcus aureus (MRSA 355) (wild type )** | **100** | **25** |
| **Staphylococcus aureus (MRSA 358) (wild type )** | **>100** | **100** |
| | | |
| **Staphylococcus epidermidis ATCC 14990** | **100** | **50** |
| | | |
| **Streptococcus pyogenes ATCC 12344** | **>100** | **100** |
| **Streptococcus pneumoniae ATCC 33400** | **50** | **25** |
| **Streptococcus pneumoniae DSM 11865 (Penr)** | **100** | **50** |
| | | |
| **Enterococcus faecalis ATCC 51299 VRE** | **100** | **50** |
| | | |
| **Enterococcus faecium (VRE) Ulm 68 (wild type )** | **>100** | **100** |
| **Enterococcus faecium (VRE) W 354 (wild type)** | **100** | **50** |
| **Enterococcus faecium (VRE) W 356 (wild type)** | **100** | **50** |
| | | |
| | | |
| **Corynebacterium amycolatum RV A2/97** | **25** | **6,25** |
| **Corynebacterium pseudodiphtheriae RV A1/95** | **12,5** | **12,5** |
| | | |
| | | |

| **gram negative bacteria** | | |
|---|---|---|
| | | |
| **E. coli ATCC 25922** | **12,5** | **6,25** |
| **E. coli ATCC 35218** | **6,25** | **3,125** |
| **E. coli (ESBL 1) UR 2884/99 (wild type)** | **12,5** | **3,125** |
| **E. coli (ESBL 3) Va 4425/01 (wild type)** | **12,5** | **6,25** |
| **E. coli (ESBI 4) Va 10866/02 (wild type)** | **12,5** | **3,125** |
| **E. coli (ESBL 9) UR 2217/03 (wild type)** | **25** | **12,5** |
| | | |
| **Klebsiella pneumoniae ATCC 13883** | **25** | **12,5** |
| **Klebsiella pneumoniae ATCC 700603 ESBL** | **100** | **25** |
| **Klebsiella pneumoniae (ESBL) CF 2 (wild type )** | **25** | **6,25** |
| **Klebsiella pneumoniae (ESBL) CF 7 (wild type)** | **50** | **25** |
| **Klebsiella pneumoniae (ESBL) CF 31 (wild type )** | **25** | **6,25** |
| | | |
| **Enterobacter cloacae ATCC 13047** | **100** | **25** |
| **Enterobacter cloacae Va 11263/03 (wild type)** | **50** | **12,5** |
| **Enterobacter cloacae Va 12270/03 (wild type)** | **12,5** | **6,25** |
| **Enterobacter aerogenes Va 12738/03 (wild type)** | **25** | **6,25** |
| | | |
| **Serratia marcescens NCTC 10211** | **100** | **50** |
| **Serratia marcescens Mero 103/013 (wild type)** | **>100** | **100** |
| **Serratia marcescens Mero 060/148 (wild type)** | **100** | **50** |
| **Serratia marcescens Mero 041/145 (wild type)** | **100** | **25** |
| | | |
| **Citrobacter freundii NCTC 9750** | **50** | **12,5** |
| **Citrobacter freundii UR 1776/03 (wild type)** | **6,25** | **1,56** |
| **Citrobacter freundii BK 2122/00 (wild type)** | **25** | **6,25** |
| **Citrobacter freundii BK 3796/00 (wild type)** | **6,25** | **3,125** |
| **Proteus mirabilis ATCC 21100** | **>100** | **50** |
| **Proteus mirabilis ATCC 9240** | **>100** | **100** |
| **Proteus mirabilis (ESBL 8) (wild type)** | **>100** | **100** |
| **Proteus mirabilis UR 1354/03 (wild type)** | **>100** | **100** |
| **Proteus mirabilis UR 1536/03 (wild type)** | **>100** | **100** |
| **Proteus mirabilis UR 1792/03 (wild type)** | **12,5** | **3,125** |
| | | |
| **Proteus vulgaris ATCC 13315** | **12,5** | **3,125** |
| **Proteus vulgaris UR 1464/03 (wild type)** | **12,5** | **3,125** |
| **Proteus vulgaris Mero 103/067 (wild type)** | **>100** | **100** |
| **Proteus vulgaris BK 8730/99 (wild type)** | **>100** | **100** |
| | | |
| **Providencia rettgeri NCTC 7475** | **>100** | **100** |
| | | |
| | | |
| **Providencia stuartii NCTC 10318** | **>100** | **100** |
| | | |
| **Morganella morganii RV B4/97** | **>100** | **100** |
| | | |
| **Aeeromonas hydrophila ATCC 7966** | **>100** | **100** |
| | | |
| **Salmonella typhimurium ATCC 13311** | **6,25** | **1,56** |
| | | |
| **Yersinia enterococcus NCTC 11174** | **>100** | **>100** |
| | | |

| **Non-fermenter** | | |
|---|---|---|
| | | |
| **Pseudomonas aeruginosa ATCC 10145** | **12,5** | **6,25** |
| **Pseudomonas aeruginosa ATCC 11440** | **12,5** | **12,5** |
| **Pseudomonas aeruginosa ATCC 11446** | **12,5** | **12,5** |
| **Pseudomonas aeruginosa ATCC 39324** | **25** | **12,5** |
| **Pseudomonas aeruginosa CF 453 Va mr (wild type)** | **25** | **12,5** |
| **Pseudomonas aeruginosa CF 479 mr (wild type )** | **25** | **12,5** |
| **Pseudomonas aeruginosa CF 509 mr (wild type)** | **25** | **12,5** |
| **Pseudomonas aeruginosa CF 645 (mukoid)( wild type)** | **12,5** | **25** |
| | | |
| **Pseudomonas fluorescens NCTC 10038** | **25** | **6,25** |
| | | |
| **Stenotrophomonas maltophilia ATCC 10257** | **25** | **6,25** |
| **Stenotrophomonas maltophilia ATCC 17666** | **50** | **6,25** |
| | | |
| **Burkholderia cepacia ATCC 25416** | **>100** | **100** |
| **Brevundimonas diminuta ATCC 11568** | **12,5** | **6,25** |
| | | |
| **Pseudomonas putida BK 4912** | **12,5** | **6,25** |
| | | |
| **Acinetobacter baumannii ATCC 19606** | **25** | **12,5** |
| **Acinetobacter genosp. 3 ATCC 19004** | **0,78** | **0,2** |
| **Acinetobacter lwoffii ATCC 15309** | **0,78** | **0,39** |
| **Acinetobacter junii ATCC 17908** | **12,5** | **3,125** |
| **Acinetobacter johnsonii ATCC 17909** | **1,56** | **0,39** |
| **Acinetobacter haemolyticus ATCC 17906** | **1,56** | **0,39** |
| **Acinetobacter calcoaceticus ATCC 23055** | **0,78** | **0,39** |
| **Acinetobacter Genosp. 10 ATCC 17924** | **1,56** | **0,39** |
| **Acinetobacter Genosp. 11 ATCC 11171** | **3,125** | **0,39** |
| | | |

| **other bacteria** | | |
|---|---|---|
| | | |
| **Propionibacterium acnes NCTC 737** | **100** | **50** |
| **Helicobacter pylori ATCC 49503** | **50** | **12,5** |
| **Campytobacterjejuni ATCC 33560** | **6,25** | **3,125** |
| | | |

| **fungi** | | |
|---|---|---|
| | | |
| **Candida albicans ATCC 10231** | **25** | **12,5** |
| **Candida albicans ATCC 24433** | **25** | **3,125** |
| **Candida albicans C.a 14 (wild type)** | **25** | **12,5** |
| **Candida albicans C.a 39 (wild type)** | **50** | **12,5** |
| **Candida albicans C.a 46 (wild type)** | **25** | **6,25** |
| | | |
| **Candida tropicalis ATCC 750** | **25** | **1,56** |
| | | |
| **Candida glabrata ATCC 90030** | **>100** | **50** |
| **Candida glabrata BK 9570/02 (wild type)** | **>100** | **>100** |
| **Candida glabrata Va 27966/02 (wild type)** | **>100** | **12,5** |
| **Candida glabrata UR 5836/02 (wild type)** | **50** | **6,25** |
| | | |
| **Candida parapsilosis ATCC 90018** | **25** | **12,5** |
| **Issatchenkia orientalis ATCC 6258** | **50** | **6,25** |
| **Cryptococcus neoformans ATCC 62066** | **12,5** | **0,78** |
| **Saccharomyces cerevisiae ATCC 9763** | **25** | **3,125** |
| | | |

| **odontogenic strains** | | |
|---|---|---|
| | | |
| **Prevotella intermedia ATCC 25611** | **3,125** | **3,125** |
| **Fusobacterium nucleatum ATCC 10953** | **3,125** | **3,125** |
| **Actinobacillus actinommycetemcomitans NCTC 10979** | **>100** | **100** |
| **Streptococcus mutans ATCC 35668** | **>100** | **100** |
| **Eikenella corrodens wild type** | **50** | **50** |

The test showed that hßD2 has antimicrobial activity against a broad spectrum of bacteria and fungi. However, the antimicrobial activity of hBD2 is more effective against gram-negative bacteria than gram-positive bacteria. This fact is reflected by the lower concentrations of the MBC and LD90 values in case of gram-negative bacteria compared with the values for gram-positive-bacteria, i.e. it is less hBD2 required to kill gram-negative bacteria. On the other hand, the antimicrobial activity of hBD2 is more effective against fungi than gram-positive bacteria.

## Claims

1. A medical device having a silane surface comprising an antimicrobial peptide, wherein the antimicrobial peptide is attached to the silane surface.

2. A medical device according to claim 1, wherein the silane is covalently bound to the medical device.

3. A medical device according to claim 1 or 2, wherein the antimicrobial peptide is covalently bound to the silane.

4. A medical device according to claim 1 or 2, wherein the antimicrobial peptide is bound to silane via Van der Waals interactions, hydrophobic interactions and/or ionic interactions.

5. A medical device according to claim 4, wherein the antimicrobial peptide is attached to the medical device via a terminal C=C, C=O, C-OH, COOH or C-NH₂ group of a silane.

6. A medical device according to any of the preceding claims, wherein the antimicrobial peptide is a member of the RNAse A super family, a defensin, cathelicidin, granulysin, histatin, psoriasin, dermicidine or hepzidine.

7. A medical device according to any of the preceding claims, wherein the antimicrobial peptide is human ß-defensin-2, human B-defensin-3 or Ribonuclease 7.

8. The medical device according to claim 6, wherein human B-defensin-2 exhibits the amino acid sequence according to the SEQ ID NO: 1 or derivatives, fragments or homologues thereof.

9. The medical device according to claim 6, wherein human ß-defensin-3 exhibits the amino acid sequence according to the SEQ ID NO: 4 or derivatives, fragments or homologues thereof.

10. The medical device according to claim 6, wherein Ribonuclease 7 exhibits the amino acid sequence according to the SEQ ID NO: 7 or derivatives, fragments or homologues thereof.

11. The medical device according to claim 9, wherein Ribonuclease 7 exhibits the amino acid sequence according to the SEQ ID NO: 10.

12. The medical device according to any of the preceding claims, wherein the silane is an alkoxysilane.

13. The medical device of claim 11, wherein the alkoxysilane is a methoxysilane.

14. The medical device according to any of the preceding claims, further comprising collagen.

15. The medical device according to claim 13, wherein collagen is attached to the self-assembled monolayer via a covalent bond or via Van der Waals interactions, hydrophobic interactions, ionic interactions and/or steric effects.
